# DEMANDE DE BREVET EUROPEEN

(11) **EP 1 705 187 A1**
(43) Date de publication de la demande: **27.09.2006**
(21) Numéro de dépôt: 06290415.6
(22) Date de dépôt: 14.03.2006
(51) Int. Cl.: C07K 14/47, A61K 8/64, C07K 9/00

(54) **Compositions cosmetiques de soin, renforcement et/ou réparation des substrats kératiniques comprenant des polpeptides KAP**

(30) Priorité: 18.03.2005 FR 0550712
(71) Demandeur: L'ORÉAL, 75008 Paris (FR)
(72) Inventeur: Barbarat, Philippe, 92270 Bois-Colombes (FR)
(74) Mandataire: Bernstein, Claire Jacqueline

(57) **Abrégé**

L'invention concerne une composition cosmétique comprenant, dans un milieu physiologiquement acceptable adapté à une application topique sur les substrats kératiniques, au moins un polypeptide de la famille des KAP (Keratin associated protein) et au moins un ingrédient cosmétique choisi parmi les agents de conditionnement, les agents de soin et/ou de traitement des substrats kératiniques et leurs mélanges.

En particulier le polypeptide de la famille des KAP possède une séquence peptidique choisie parmi (i) les séquences SEQ ID N° 1 à SEQ ID N°66, (ii) un fragment desdites séquences comprenant au moins 3 acides aminés consécutifs d'une séquence répétée choisie parmi la séquence SZCCXPSCCXP (Z : ⌀, P et X : Q,V,R,I) et la séquence YGGXGYGSGY (X :Y,L,F) et (iii) leurs homologues.

L'invention porte également sur un procédé cosmétique de soin, de renforcement et/ou de réparation des substrats kératiniques, en particulier des fibres kératiniques, mettant en oeuvre lesdites compositions.

## Description

Le domaine de l'invention concerne le soin, le renforcement et/ou la réparation des substrats kératiniques, en particulier des fibres kératiniques.

L'invention porte notamment sur des compositions comprenant, dans un milieu physiologiquement acceptable adapté à une application topique sur les substrats kératiniques, au moins un polypeptide de la famille des KAP et au moins un ingrédient cosmétique choisi parmi les agents de conditionnement, les agents de soin et/ou de traitement des substrats kératiniques et leurs mélanges.

Elle concerne également un procédé cosmétique de soin, de renforcement et/ou de réparation des substrats kératiniques, en particulier des fibres kératiniques, mettant en oeuvre lesdites compositions.

L'invention porte également sur l'utilisation cosmétique d'au moins un polypeptide KAP dans une composition, comme agent destiné à maintenir et/ou restaurer et/ou améliorer le contenu protéique du substrat kératinique et par là-même conserver et/ou restaurer et/ou améliorer les propriétés physiques et/ou mécaniques des substrats kératiniques.

Le terme "substrat kératinique" selon l'invention englobe la peau, les ongles et les fibres kératiniques. On entend par "fibres kératiniques", les cheveux, les cils, les sourcils et les poils.

Les kératines sont des composés fondamentaux de la peau, du cheveu, du cil et de l'ongle. Ces protéines fibreuses, insolubles dans l'eau, participent notamment à leur forme, à leur élasticité et à leur résistance.

Dans le cheveu par exemple, il existe deux grands types de protéines :
- les kératines α, divisées notamment en deux groupes -les kératines acides (hHa1-8) et les kératines basiques à neutres (hHb1-6)- sont de longues chaînes polypeptidiques hélicoïdales superenroulées en hélices α qui forment les microfibrilles ou filaments intermédiaires du cortex (FI), insérés dans une matrice de KAPs ;
- les KAP (Keratin Associated Proteins) ou IFAPs (Intermediate Filaments Associated Protein) forment la matrice amorphe entre ces microfibrilles. Il existe 3 types de KAPs réparties en 23 familles à ce jour : les KAP très riches en cystéine (UHS ou Ultra High Sulfur qui contiennent plus de 30 mol% de cystéine), les KAP riches en cystéine (HS ou High Sulfur qui contiennent moins de 30 mol% de cystéine) et les KAP riches en glycine et tyrosine (HGT ou High Glycine and Tyrosine).

L'ensemble de ces protéines assure, par le biais de liaisons covalentes (ponts disulfures notamment), liaisons salines, liaisons hydrogène ou liaisons hydrophobes entre les chaînes d'acides aminés, la cohésion de la structure tridimensionnelle du cheveu. En particulier, les séquences des kératines α et des KAPs présentent des motifs répétés, pour les uns riches en résidus lysine, pour les autres en résidus glutamine, qui constituent des substrats potentiels d'enzymes (ex : transglutaminases) aptes à former des ponts interfilaments (FI-FI), inter KAPs (KAP-KAP), ou des laisons covalentes entre les filaments de kératines et les KPAs (FI-KAP), jouant un rôle essentiel dans la structure et la forme de la tige pilaire.

Les substrats kératiniques et les fibres kératiniques en particulier, peuvent être sensibilisés ou affaiblis par l'action chimique d'agents atmosphériques (UV, pollution...), et/ou de traitements (décoloration, coloration, défrisage, permanente...).
On sait par exemple que les cheveux sensibilisés ou fragilisés sont souvent secs, rêches, difficiles à démêler et à coiffer.
On sait aussi que les ongles présentent, de façon fréquente, des défauts de structure et de consistance, qui ont pour effet de rendre la surface des ongles inesthétique, ce qui peut être source de gêne et de désagréments multiples.

On connaît de la demande WO 99/49837 L'OREAL l'utilisation de dérivés de polyaminoacides dans le renforcement et le soin des fibres kératiniques.
La demande WO 03/042387 décrit des séquences nucléotidiques et polypeptidiques de type KAP à activité de liaison à la kératine du cheveu, ainsi que des agents cosmétiques ou thérapeutiques intégrant lesdites séquences.

Mais à la connaissance de la Demanderesse, il n'a jamais été décrit ni suggéré des compositions cosmétiques comprenant au moins un polypeptide KAP et au moins un ingrédient cosmétique choisi parmi les agents de conditionnement, les agents de soin et/ou de traitement des substrats kératiniques et leurs mélanges, ladite association étant destinée notamment à améliorer l'aspect et/ou l'état de surface des substrats kératiniques, à préserver et/ou améliorer les propriétés physiques et/ou mécaniques des substrats kératiniques, en particulier lorsque les substrats kératiniques sont sensibilisés ou affaiblis par l'action chimique d'agents atmosphériques et/ou de traitements.

L'invention concerne donc une composition cosmétique comprenant, dans un milieu physiologiquement acceptable adapté à une application topique sur les substrats kératiniques, au moins un polypeptide de la famille des KAP et au moins ingrédient cosmétique choisi parmi les agents de conditionnement, les agents de soin et/ou de traitement des substrats kératiniques et leurs mélanges.

En particulier, le polypeptide de la famille des KAP utilisable dans les compositions de l'invention possède une séquence peptidique choisie parmi (i) les séquences SEQ ID N° 1 à SEQ ID N°66, (ii) un fragment desdites séquences comprenant au moins 3 acides aminés consécutifs d'une séquence répétée choisie parmi la séquence SZCCXPSCCXP (Z: ∅, P et X: Q,V,R,I) et la séquence YGGXGYGSGY (X :Y,L,F) et (iii) leurs homologues. 'Z' peut correspondre à un acide aminé proline (P) ou aucun acide aminé (∅).

Les SEQ ID N°1 à SEQ ID N°66 sont présentées en annexe.

Les fragments (ii) comprenant au moins 3 acides aminés consécutifs d'une séquence répétée choisie parmi la séquence SZCCXPSCCXP (Z : ∅, P et X : Q,V,R,I) et la séquence YGGXGYGSGY (X :Y,L,F) seront avantageusement des substrats potentiels d'enzymes (ex : transglutaminases) aptes à former des ponts interfilaments (FI-FI), inter KAPs (KAP-KAP), ou des liaisons covalentes entre les filaments de kératines et les KAPs (FI-KAP).
En particulier, ledit fragment constitué d'au moins 3 acides aminés consécutifs de ladite séquence répétée SZCCXPSCCXP (Z: ∅, P et X: Q,V,R,I) et la séquence YGGXGYGSGY (X :Y,L,F), de préférence d'au moins 4 acides aminés, et encore plus préférentiellement d'au moins 5 acides aminés consécutifs de ladite séquence répétée, comprendra au moins un acide aminé apte à former des liaisons covalentes (ex : cystine), liaisons salines (ex : lysine, arginine, histidine, aspartate, glutamate), liaisons hydrogène (ex : sérine, tyrosine) ou liaisons hydrophobes (ex : glycine, alanine, valine, leucine, isoleucine), soit avec les filaments intermédiaires de kératine (FI), soit avec d'autres KAPs ou encore avec d'autres protéines constitutives du cheveu.

Ledit fragment (ii) pourra de préférence contenir de 3 à 60 acides aminés, en particulier de 3 à 20 acides aminés, et encore plus préférentiellement de 3 à 10 acides aminés. Ce fragment de polypeptide peut être obtenu par protéolyse ou de manière synthétique selon les méthodes connues.

Selon un mode particulier, on pourra utiliser la séquence SEQ ID N°9 ou de préférence un fragment de cette séquence SEQ ID N°9, en particulier le fragment SPCCR.

Par 'homologues', on entend selon l'invention toute séquence peptidique identique à au moins 50%, de préférence à au moins 80% et encore plus préférentiellement à au moins 95 % de ladite séquence peptidique (i) choisie parmi les séquences SEQ ID N° 1 à SEQ ID N°66 ou dudit fragment (ii) tels que définis précédemment, chez la même espèce ou chez une espèce différente ; dans ce dernier cas, on le désigne également par 'polypeptide orthologue'.
Et par 'pourcentage d'identité' entre deux séquences peptidiques ou séquences d'acides aminés, on entend désigner un pourcentage de résidus d'acides aminés identiques entre les deux séquences à comparer, obtenu après le meilleur alignement, c'est-à-dire l'alignement optimal réalisé par exemple au moyen de l'algorithme d'homologie locale de Smith et Waterman (1981, Ad. App. Math. 2 :482), au moyen de l'algorithme d'homologie locale de Neddleman et Wunsch (1970, J.Mol. Biol. 48 : 443), au moyen de la méthode de recherche de similarité de Pearson et Lipman (1988, Proc. Natl. Acad. Sci. USA 85 :2444), au moyen de logiciels informatiques utilisant ces algorithmes (GAP, BESTFIT, BLAST P, BLAST N disponible sur le site NCBI, FASTA et TFASTA dans le Wisconsin Genetics Software Package, Genetics Computer Group, 575 Science Dr, Madison, WI).

Les polypeptides KAP selon l'invention peuvent être des polypeptides d'origine naturelle ou synthétique.
Par 'origine naturelle', on entend un polypeptide à l'état pur ou en solution à différentes concentrations, obtenu par différents procédés d'extraction à partir d'une matière kératinique (peau, ongle, cheveu, en particulier cheveu) d'origine naturelle.
Par 'origine synthétique', on entend un polypeptide à l'état pur ou en solution à différentes concentrations, obtenu chimiquement ou par production dans un organisme après introduction dans cet organisme des éléments nécessaires à cette production. Par exemple, le polypeptide sera un polypeptide recombinant.

Le polypeptide de la famille des KAP est présent dans la composition en une quantité suffisante pour obtenir l'effet recherché, à savoir maintenir et/ou restaurer et/ou améliorer le contenu en protéine des substrats kératiniques. En particulier cette quantité pourra aller de 0,001 à 30% en poids par rapport au poids total de la composition, de préférence de 0,01 à 15% et encore plus préférentiellement de 0,1 à 5% en poids par rapport au poids total de la composition.

Par 'agent de conditionnement des substrats kératiniques', on entend notamment selon l'invention les huiles, les cires, les céramides, les gommes, les silicones, les polysiloxanes, les polymères filmogènes ou les polymères fixants et leurs mélanges.
Cet agent sera notamment destiné à améliorer l'aspect et/ou l'état de surface des substrats kératiniques (ex : cheveux plus doux, plus lisses, moins fourchus, ongles plus brillants...), à préserver et/ou améliorer les propriétés physiques et/ou mécaniques des substrats kératiniques (ex : cheveux plus forts, plus faciles à coiffer, ongles moins cassants...), en particulier lorsque les substrats kératiniques sont sensibilisés ou affaiblis par l'action chimique d'agents atmosphériques et/ou de traitements.

Comme huiles, on peut utiliser par exemple les huiles naturelles hydrogénées ou non, les huiles synthétiques hydrocarbonées, cycliques ou aliphatiques, linéaires ou ramifiées, saturées ou insaturées, telles que par exemples les poly α-oléfines, en particulier les polydécènes et polyisobutènes, les huiles de silicone volatiles ou non, organo-modifiées ou non, solubles ou non, les huiles fluorées ou perfluorées, les esters gras, les esters d'alcools polyhydriques et les glycérides.
A titre d'exemples, on peut citer les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le düsostéarylmalate, le citrate de trüsocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.
Ces huiles peuvent être présentes en une teneur allant de 0,01 à 90 %, et mieux de 0,1 à 85 % en poids, par rapport au poids total de la composition.

On peut également utiliser des cires synthétiques ou naturelles d'origine végétale, minérale, animale, ayant un point de fusion supérieur ou égal à 30 °C pouvant aller jusqu'à 120 °C. Ces cires peuvent être hydrocarbonées, fluorées et/ou siliconées. A titre d'exemples, on peut citer la cire d'abeilles, la cire de Carnauba ou de Candellila, la paraffine, les cires microcristallines, la cérésine ou l'ozokérite; les cires synthétiques comme les cires de polyéthylène ou de Fischer Tropsch, les cires de silicones comme les alkyl ou alkoxy-diméticone ayant de 16 à 45 atomes de carbone et les céramides.

On peut également utiliser des gommes et résines de silicone, des protéines ou des hydrolysats de protéines quaternisés ou non ou un mélange de ces divers agents. On peut également citer des silicones volatiles ou non, linéaires ou cycliques et leurs mélanges, les polydiméthylsiloxanes, les polyorganosiloxanes quaternisés, les polyorganosiloxanes à groupements aminoalkyles modifiés par des groupements alcoxycarbonyalkyles, des polyorganosiloxanes tels que le copolymère polydiméthylsiloxane-polyoxyalkyle du type Diméthicone Copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne, des polysiloxanes organo modifiés par des groupements mercapto ou mercaptoalkyles tels que ceux décrits dans le brevet français FR 1 530 369 et dans la demande de brevet européen EP 295 780, ainsi que des silanes tels que le stéaroxytriméthylsilane.

Comme polymères filmogènes, on peut citer les polymères synthétiques, de type radicalaire ou de type polycondensat, les polymères d'origine naturelle et leurs mélanges. En particulier, on peut citer les polymères acryliques, les polyuréthanes, les polyesters, les polyamides, les polyurées, les polymères cellulosiques comme la nitrocellulose. En milieu aqueux, on utilisera préférentiellement les polyuréthanes, par exemple anioniques, les polyesters-polyuréthanes, les polyéther-polyuréthanes, les polymères radicalaires, notamment de type acrylique, acrylique styrène et/ou vinylique, les polyesters, les résines alkydes, seuls ou en mélange.

Comme polymères fixants, utilisables notamment dans les compositions de mise en forme et/ou de coiffage et/ou de maintien des cheveux, on peut citer les polymères fixants anioniques, les polymères fixants cationiques, les polymères fixants non ioniques, les polymères fixants amphotères et leurs mélanges.
Comme exemple de polymères fixants anioniques, on peut citer ceux à groupements carboxyliques, tels que les homo- ou copolymères d'acide acrylique ou méthacrylique ou leurs sels, les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés, les copolymères d'acide crotonique, les copolymères d'acides ou d'anhydrides carboxyliques monoinsaturés en C₄-C₈, les polyacrylamides comportant des groupements carboxylates.
Comme exemples de polymères fixants cationiques, on peut citer les copolymères d'acrylamide et de méthacrylate de diméthylaminoéthyle quatemisé au sulfate de diméthyle ou avec un halogénure de diméthyle ; les polysaccharides cationiques, de préférence à ammonium quaternaire tel que les gommes de guar contenant des groupements cationiques trialkylammonium; les copolymères quaternaires de vinylpyrrolidone et de vinylimidazole ; les chitosanes ou leurs sels; les sels utilisables sont en particulier les acétate, lactate, glutamate, gluconate ou le pyrrolidone-carboxylate de chitosane ; les dérivés de cellulose cationiques tels que les copolymères de cellulose ou de dérivés de cellulose greffés avec un monomère hydrosoluble comportant un ammonium quaternaire.
Comme polymères fixants amphotères , on peut citer les copolymères à motifs vinyliques acides et à motifs vinyliques basiques.

Par 'agent de soin et/ou de traitement des matières kératiniques', on entend notamment les actifs lipophiles ou hydrophiles utilisés dans les compositions de soin et/ou de traitement de la peau, des cheveux, des cils et des ongles.
Ces agents seront notamment destinés à améliorer l'aspect et/ou l'état de surface des substrats kératiniques, à préserver et/ou améliorer les propriétés physiques et/ou mécaniques des substrats kératiniques, en particulier lorsque les substrats kératiniques sont sensibilisés ou affaiblis par l'action chimique d'agents atmosphériques et/ou de traitements.

Pour le soin et/ou le traitement de la peau, on peut citer notamment les agents desquamants ou hydratants, les agents dépigmentants ou propigmentants, les agents stimulant la synthèse de macromolécules dermiques ou épidermiques et/ou empêchant leur dégradation, les agents anti-glycation, les agents inhibiteurs de NO-synthase, les agents anti-pollution ou antiradicalaires, les agents apaisants, les agents agissant sur la microcirculation...

### Agents desquamants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

### Agents hydratants

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides comme le produit commercialisé sous la référence Pentavitin, le miel, les alginates (notamment le produit Sobalg PH 154 commercialisé par la société Grindsted), les carbonates cycliques, l'acide N-lauroyl pyrrolidone carboxylique ou ses sels, notamment le sel de sodium commercialisé sous la référence Nalidone, et la N-α-benzoyl-L-arginine ;
- soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés.
   Ces composés peuvent représenter de 0,001% à 30%, et de préférence de 0,01 à 20%, du poids total de la composition selon l'invention.

### Agents dépigmentants, anti-pigmentants ou pro-pigmentants

Les agents dépigmentants ou anti-pigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.
Comme agent pro-pigmentant, on peut citer l'extrait de pimprenelle (Sanguisorba officinalis) commercialisé par la société MARUZEN et les extraits de chrysanthème (Chrysanthemum morifolium).

### Agents stimulant la synthèse de macromolécules dermiques ou épidermiques et/ou empêchant leur dégradation

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9. On peut citer: les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB® ), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl® ; les héparinoïdes ; et les pseudodipeptides tels que l'acide {2-[acétyl-(3-trif)uorométhy)-phény))-amino]-3-méthy)-butyry)amino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G® .

### Agents anti-plycation

Par "agent anti-glycation", on entend un composé prévenant et/ou diminuant la glycation des protéines de la peau, en particulier des protéines du derme telles que le collagène. Des exemples d'agents anti-glycation sont les extraits végétaux de la famille des Ericaceae, tels qu'un extrait de myrtille *(Vaccinium angusfifollium) ;* l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 2 802 425, FR 2 810 548, FR 2 796 278 et FR 2 802 420, respectivement. Le resvératrol est particulièrement préféré pour une utilisation dans cette invention.

### Inhibiteurs de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect® , ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

### Agents anti-pollution ou anti-radicalaires

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F® , la fraction hydrosoluble de maïs commercialisée par la société SOLABIA sous la dénomination commerciale Phytovityl® , le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect® .

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl® .

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl® .

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; et la mélatonine.

### Agents apaisants

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, les extraits de plantes telles que *Paeonia suffruticosa* et / ou *lactitlora, Laminaria saccharina, Boswellia serrata, Centipeda cunnighami, Helianthus annuus, Linum usitatissimum, Cola nitida, Epilobium Angustifolium, Aloe vera, Bacopa monieri,* les sels de l'acide salicylique et en particulier le salicylate de zinc, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'ecchium, ou de poisson, des extraits de plancton, la capryloyl glycine, le Seppicalm VG (sodium palmitoylproline et nymphea alba) de Seppic, les tocotrienols, le piperonal, un extrait de clou de girofle, les phytostérols, la cortisone, l'hydrocortisone, l'indométhacine et la beta méthasone.

### Agents agissant sur la microcirculation

Les actifs agissant sur la microcirculation (vasoprotecteur ou vasodilatateur) peuvent être choisis parmi les flavonoïdes, les ruscogénines, les esculosides, l'escine extraite du marron d'Inde, les nicotinates, l'héperidine méthyl chalcone, les huiles essentielles de lavande ou de romarin, les extraits de Ammi Visnaga.

Pour le soin et/ou le traitement des cheveux, on peut citer par exemple les agents hydratants, les agents adoucissants, les agents anti-radicalaires, les vitamines, les acides aminés, les protéines, les agents anti-pelliculaires, les agents anti-séborrhéiques, les agents réducteurs (pour la mise en forme ou la déformation permanente des cheveux), les précurseurs de colorants capillaires, les colorants capillaires directs, les agents oxydants...

### Agents antipelliculaires

Les agents antipelliculaires peuvent être tout agent actif utile pour prévenir l'apparition des pellicules, diminuer leur nombre et/ou les faire disparaître totalement. Ainsi, l'agent antipelliculaire peut être choisi parmi les agents antifongiques et/ou antibactériens.

Les agents antipelliculaires utilisables selon l'invention sont notamment choisis parmi :
1) les sels de pyridinethione notamment les sels de calcium, de magnésium, de barium, de strontium, de zinc, de cadmium, d'étain et de zirconium. Le sel de zinc de pyridinethione est notamment commercialisé sous la dénomination Omadine de zinc par la société OLIN.
2) les dérivés de 1-hydroxy-2-pyrrolidone, tels que par exemple le 1-hydroxy-4-méthyl-2-pyridone, le 1-hydroxy-6-méthyl-2-pyridone, le 1-hydroxy-4,6-diméthyl-2-pyridone, le 1-hydroxy-4-méthyl-6-(2,4,4-triméthylpentyl)-2-pyridone, le 1-hydroxy-4-méthyl-6-cyclohexyl-2-pyridone, le 1-hydroxy-4-méthyl-6-(méthyl-cyclohexyl)-2-pyridone, le 1-hydroxy-4-méthyl-6-(2-bicyclo[2,2,1]hepty))-2-pyridone, le 1-hydroxy-4-methyl-6-(4-methyl-phenyl)-2-pyridone, le 1-hydroxy-4-methyl-6-[1-(4-nitrophenoxy)-butyl]-2-pyridone, le 1-hydroxy-4-méthyl-6-(4-cyanophenoxymethyl)-2-pyridone, le 1-hydroxy-4-methyl-6-(phenylsulfonylmethyl)-2-pyridone, le 1-hydroxy-4méthyl-6-(4-bromo-benzyl)-2-pyridone. Ces composés peuvent être utilisés sous forme de sels avec des bases organiques ou inorganiques. Un composé préféré est I' Octopirox (1-hydroxy-4-méthyl-6-(2,4,4-triméthylpentyl)-2-pyridone, sel de monoéthanolamine) commercialisé par la société HOECHST.
3) le 2,2'-dithio-bis-(pyridine-N-oxide) sous forme de sels inorganiques, dont le sulfate de magnésium ;
4) les trihalogéno carbamide ;
5) le triclosan ;
6) les composés azolés tels que le climbazole, le kétoconazole, le clotrinazole, l'econazole, l'isoconazole et le miconazole ;
7) les polymères antifongiques tels que l'amphotéricine B ou la nystatine ;
8) le sulfure de sélénium ;
9) d'autres agents antipelliculaires, sont le soufre sous ses différentes formes, le sulfure de cadmium, l'allantoïne, les goudrons de houille ou de bois et leurs dérivés en particulier l'huile de cade, l'acide salicylique, l'acide undécylénique, l'acide fumarique, les allylamines telle que la terbinafine.

### Agents anti-séborrhéiques

Lorsque la composition selon l'invention comprend un agent anti-séborrhéique tel qu'un inhibiteur de 5α-réductase, celui-ci peut notamment être choisi parmi :
- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5® ;
- un extrait de Laminaria saccharina commercialisé notamment par la société SECMA sous la dénomination commerciale Phlorogine® ;
- un extrait de Spiraea ulmaria commercialisé notamment par la société SILAB sous la dénomination commerciale Sebonormine® ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED ;
- des extraits de plantes du genre Silybum ; et
- des extraits d'Eugenia caryophyllata contenant de l'eugénol et du glucoside d'eugényle.

### Agents réducteurs

Ces agents utilisables notamment dans les compositions de mise en forme ou de déformation permanente des cheveux, peuvent être choisis par exemple parmi l'acide thioglycolique, le monothioglycolate de glycérol ou de glycol, la cystéamine et ses dérivés acylés en C1-C4 tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide bêta-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)o-hydroxyalkylamides décrits dans la demande de brevet EP 354 835 et les N-mono- ou N,N-dialkylmercapto 4-butyramides décrits dans la demande de brevet EP 368763, les aminomercaptoalkylamides décrits dans la demande de brevet EP 403 267 et les alkylaminomercaptoalkylamides décrits dans la demande de brevet EP 432000.
Lorsque les compositions selon l'invention contiennent au moins un agent réducteur, celui-ci est avantageusement présent à une concentration maximale de 20% en poids, et de préférence, comprise entre 0,1 et 10% en poids par rapport au poids total de la composition.

### Précurseurs de colorants capillaires

On peut citer notamment les colorants d'oxydation qui sont généralement choisis parmi les bases d'oxydation, les orthodiphénols, les coupleurs, et leurs mélanges.
Les bases d'oxydation, de type para ou ortho, sont des composés qui ne sont pas des colorants en eux-mêmes, mais forment un colorant par un processus de condensation oxydative, soit sur eux-mêmes, soit en présence d'un coupleur ou modificateur. Ils comportent des groupements fonctionnels, soit deux groupements amino, soit un groupement amino et un groupement hydroxy en position para ou ortho l'un par rapport à l'autre. Elles peuvent notamment être choisies parmi les ortho- et paraphénylènediamines, les bases doubles, les ortho- et para-aminophénols, les bases hétérocycliques ainsi que les sels d'addition de tous ces composés avec un acide.

Les orthodiphénols sont des composés comportant au moins un cycle aromatique dont au moins deux carbones consécutifs portent un groupe hydroxyle. De préférence, le cycle aromatique est un cycle benzénique ou un cycle aromatique condensé.
Le cycle aromatique peut être un cycle aromatique condensé contenant éventuellement un ou plusieurs hétéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Une autre classe de colorants d'oxydation est constituée par les coupleurs, comme expliqué précédemment. Les coupleurs peuvent être choisis parmi ceux classiquement utilisés en teinture d'oxydation et notamment parmi les méta-aminophénols, les métaphénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide, ces composés étant différents des composés orthodihydroxylés de l'invention.
Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

### Colorants directs

Parmi ces colorants, utilisés notamment pour modifier les nuances en les enrichissant de reflets, on peut citer les colorants nitrés, azoïques ou anthraquinoniques, neutres, cationiques ou anioniques dans la proportion pondérale d'environ 0,001 à 20% et de préférence de 0,01 à 10% en poids par rapport au poids total de la composition.

### Agents oxydants

Parmi les agents oxydants classiquement utilisés, on peut citer le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés. L'agent oxydant est choisi de préférence dans le groupe formé par l'eau oxygénée, le peroxyde d'urée, les persels tels que les perborates ou les persulfates ou leurs mélanges.

Pour le soin et/ou le traitement des cils, on peut citer notamment des agents favorisant le recourbement des cils, tels que des polymères filmogènes (ex : les polymères acryliques, les polyuréthanes, les polyesters, les polyamides, les polyurées, les polymères cellulosiques comme la nitrocellulose).

Pour le soin et/ou le traitement des ongles, on peut citer notamment des agents favorisant la pousse des ongles, des agents durcisseurs d'ongles...
On peut citer notamment un extrait de bactérie filamenteuse non synthétique tel que défini dans la demande EP 1 479 367 (L'OREAL). En particulier il s'agira d'un extrait de *Vitreoscilla filiformis.*

L'homme du métier adaptera la quantité d'ingrédient cosmétique dans la composition en fonction de l'effet recherché et de façon telle que la quantité d'ingrédient cosmétique présente dans la composition n'altère pas les propriétés recherchées du polypeptide KAP.

En particulier, le(s)dit(s) ingrédient(s) cosmétique(s) sera présent dans la composition en une quantité pouvant aller de 0,001 à 20% en poids par rapport au poids total de la composition, de préférence de 0,01 à 10% et encore plus préférentiellement de 0,1 à 2% en poids par rapport au poids total de la composition.

La composition pourra comprendre en outre des adjuvants cosmétiques et/ou des agents de formulation classiquement utilisés dans les compositions cosmétiques, tels que les solvants organiques, les agents tensioactifs, les agents plastifiants, les agents épaississants, les agents émulsionnants, les conservateurs, les filtres UV, les charges, les matières colorantes, les parfums, les agents opacifiants, les agents humectants, les agents d'ajustement et de fixation du pH, les agents anti-bactériens, les agents antifongiques, les agents propulseurs et leurs mélanges.

Comme solvants organiques, on peut citer les solvants organiques hydrophiles, les solvants organiques lipophiles, les solvants amphiphiles ou leurs mélanges. Parmi les solvants organiques hydrophiles, on peut citer par exemple des mono-alcools inférieurs, linéaires ou ramifiés, ayant de 1 à 8 atomes de carbone comme l'éthanol, le propanol, le butanol, l'isopropanol, l'isobutanol, des polyéthylèneglycols éventuellement oxyéthylénés, des polyols tels que le propylèneglycol, l'isoprèneglycol, le butylène glycol, le glycérol, le sorbitol et ses dérivés, les éthers de glycol et les éthers de propylène glycol. Comme solvants organiques amphiphiles, on peut citer des polyols tels que des dérivés de propylèneglycol. Comme solvants organiques lipophiles, on peut citer par exemple les esters gras.

On peut également citer les cétones liquides à température ambiante tels que méthyléthylcétone, méthylisobutylcétone, düsobutylcétone, l'isophorone, la cyclohexanone, l'acétone ; les éthers de propylène glycol liquides à température ambiante ; les esters à chaîne courte (ayant de 3 à 8 atomes de carbone au total); les éthers liquides à température ambiante ; les alcanes liquides à température ambiante ; les composés cycliques aromatiques liquides à température ambiante ; les aldéhydes liquides à température ambiante et leurs mélanges.
Ces solvants peuvent être généralement présents en une teneur allant de 0 à 90 % en poids par rapport au poids total de la composition, de préférence de 0,1 à 90%, de préférence encore de 10 à 90%, et mieux de 30 à 90 % en poids par rapport au poids total de la composition.

Comme agents tensioactifs, on peut citer un agent tensioactif de type non-ionique, anionique, cationique ou amphotère. En particulier pour les compositions capillaires, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Comme agents plastifiants, on peut citer les glycols et leurs dérivés, les esters de glycol, les dérivés de propylène glycol, les esters d'acides carboxyliques, les dérivés oxyéthylénés et leurs mélanges. La teneur en plastifiant peut, par exemple, aller de 0, 1 % à 15% en poids par rapport au poids total de la composition, et de préférence de 0,5 à 10% en poids.

Comme agents épaississants, on peut citer la cellulose et ses dérivés comme les éthers de cellulose, les hétérobiopolysaccharides tels que la gomme de xanthane, les scléroglucanes, les acides polyacryliques réticulés ou non. On peut citer par exemple les polymères naturels tels que les gommes de xanthane et de guar ou encore les dérivés cellulosiques, les amidons et les alginates ; les polymères synthétiques acryliques réticulés tels que les Carbopols® commercialisés par la société Goodrich et les polymères d'acide 2-acrylamido-2-méthylpropane-sulfonique (AMPS) réticulés et au moins partiellement neutralisés, comme par exemple le produit commercialisé sous la dénomination Hostacerin® AMPS par la société Clariant. Les agents épaississants sont présents de préférence dans des proportions variant entre 0,1 et 5 % en poids environ par rapport au poids total de la composition.

Comme agents émulsionnants, on peut citer entre autres le laurylsulfate, le stéarate de triéthanolamine, ou les alcools gras tels que l'alcool stéarylique ou l'alcool cétylique.
Parmi les agents émulsionnants susceptibles d'être utilisés dans les compositions sous forme d'émulsion eau-dans-l'huile, on peut citer entre autres les esters de glycérol, les alcools éthoxylés, la lanoline, l'alcool de lanoline, le cholestérol et les divers oléates de sorbitan. Les agents émulsionnants sont généralement présents en une proportion comprise entre 1 et 10 % en poids par rapport au poids total de la composition.
Parmi les émulsionnants des compositions sous forme de gels, on peut notamment citer l'éther polyoxyéthylé et l'alcool oléylique ou son ester phosphonique, l'alcool laurylique polyéthoxylé, l'alcool oléylique polyéthoxylé, l'alcool cétylique oxyéthylé, et divers polyoxyéthylène glycols d'acides gras.

Comme matières colorantes, on peut citer les colorants hydrosolubles, et les substrats colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.
Par 'pigments', il faut comprendre des particules de toute forme, blanches ou colorées, minérales ou organiques, insolubles dans le milieu physiologique, destinées à colorer la composition.
Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.
Par 'nacres', il faut comprendre des particules de toute forme irisées, notamment produites par certains mollusques dans leur coquille ou bien synthétisées.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.

Les paillettes peuvent être choisies parmi celles en résine acrylique, polyester, polyéthylène téréphtalate, en aluminium.

Comme charges, on peut citer notamment les charges minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon® ) (Orgasol® de chez Atochem), de poly-β-alanine et de polyéthylène, les poudres de polymères de tétrafluoroéthylène (Téflon® ), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium. Elles sont généralement présentes en une teneur allant de 0,01 % à 50 % en poids, par rapport au poids total de la composition, de préférence allant de 0,01 % à 30 % en poids.

Comme filtres UV ou agents photoprotecteurs, on peut citer les filtres UV organiques ou les filtres UV minéraux (oxydes de zinc ou de titane).
Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges. Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.
Les filtres sont généralement présents dans les compositions selon l'invention dans des proportions allant de 0,1 à 20% en poids par rapport au poids total de la composition, et de préférence allant de 0,2 à 15% en poids par rapport au poids total de la composition.

Comme conservateurs, on peut citer les esters d'acide para-hydroxybenzoïque, l'octane 1,2-diol, l'iodo-3 propynyl-2 butyl carbamate, le phénoxyéthanol et le gluconate de chlorhexidine.

Comme agents propulseurs utilisés de préférence dans les compositions de mousse ou de laque pour cheveux, on peut citer par exemple l'air comprimé, le dioxyde de carbone, le protoxyde d'azote, l'azote, l'oxyde nitreux, le diméthyléther, les hydrocarbures aliphatiques liquéfiables tels que le propane, les butanes dont l'isobutane, le pentane, l'isopentane, le néopentane, et leurs mélanges, les hydrocarbures chlorés et/ou fluorés ou leurs mélanges. Parmi les hydrocarbures chlorés et/ou fluorés, on peut citer le monochlorodifluorométhane, le dichlorodifluorométhane, le mono-chlorodifluoroéthane le 1,1-difluoroéthane, et le dichloro-tétrafluoroéthane, ainsi que leurs mélanges et en particulier le mélange de monochlorodifluorométhane-monochlorodifluoroéthane (40/60) ainsi que le mélange de dichlorodifluorométhane-dichlorotétra-fluoroéthane (60/40).
La proportion en agent propulseur utilisée n'est pas critique mais elle détermine la densité de la mousse produite. La proportion en agent propulseur est généralement comprise entre 1 et 20 % en poids par rapport au poids total de la composition aérosol, et de préférence entre 5 et 15 %.

Les compositions selon l'invention comprenant, dans un milieu physiologiquement acceptable adapté à une application sur les substrats kératiniques, au moins un polypeptide KAP et au moins un ingrédient cosmétique tel que défini précédemment, pourront se présenter sous toutes les formes galéniques connues pour être adaptées à une application topique sur les substrats kératiniques (peau, cils, ongles, cheveux).

Par "milieu physiologiquement acceptable", on entend selon l'invention un milieu cosmétiquement acceptable et compatible avec les substrats kératiniques.
D'une manière générale, un milieu compatible avec les substrats kératiniques peut être anhydre ou aqueux ; il peut ainsi comprendre une phase aqueuse et/ou une phase grasse.

Par phase aqueuse, on entend de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en C₂ et des aldéhydes en C₂-C₄ hydrophiles. L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

Par phase grasse, on entend une phase comprenant au moins un corps gras, choisi parmi les huiles, les cires, les corps gras pâteux et leurs mélanges.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions adaptées à une application topique selon l'invention peuvent se présenter notamment sous forme de solution aqueuse, hydroalcoolique ou huileuse, d'émulsions huile-dans-eau (H/E) ou eau-dans-huile (E/H) ou multiple (E/H/E ou H/E/H), de gel aqueux ou huileux, de produits anhydres déshydratés, ou de dispersions d'une phase huileuse dans une phase aqueuse à l'aide de sphérules, ces sphérules pouvant être des nanoparticules polymériques telles que des nanosphères et les nanocapsules, ou des vésicules lipidiques de type ionique (liposomes) et/ou non ionique.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Pour une application sur les cheveux en particulier, la composition pourra se présenter sous la forme de crèmes, de lotions, de gels, d'émulsions, de mousses ou sous la forme de compositions pour aérosol comprenant également un agent propulseur sous pression. Elle peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant, mousse), d'un shampoing colorant, d'une composition de permanente, d'un masque traitant, d'une crème ou d'un gel moussant de nettoyage des cheveux. Elle peut encore se présenter sous forme de teinture ou de mascara capillaire à appliquer au pinceau ou au peigne.

Pour une application sur les cils, la composition se présentera de préférence sous la forme d'un mascara pouvant être une composition de maquillage des cils, une base de maquillage des cils, une composition à appliquer sur un mascara, dite encore top-coat, ou bien encore une composition de traitement cosmétique des cils. Le mascara est plus particulièrement destiné aux cils d'êtres humains, mais également aux faux-cils.

Pour une application sur les ongles, la composition pourra être un produit de maquillage des ongles tel qu'un vernis à ongles coloré, une base pour les ongles ou "base-coat" en terminologie anglo-saxonne, une composition de finition, encore appelée "top-coat", à appliquer sous ou sur le produit de maquillage des ongles, un dissolvant pour vernis à ongles ou bien encore un produit de soin cosmétique des ongles tel qu'une base traitante destinée à protéger, à renforcer et/ou réparer les ongles.

Pour une application sur la peau, la composition pourra être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage pour la peau.

L'application desdites compositions sera réalisée par tout moyen adéquat, tel qu'un pinceau, une brosse, un spray ou les doigts par exemple.

L'invention concerne également un procédé cosmétique de soin, de traitement, de renforcement et/ou de réparation des substrats kératiniques, caractérisé en ce que l'on applique sur la peau, les cheveux, les cils ou les ongles une composition telle que définie précédemment, suivi éventuellement d'un rinçage.

Par 'soin des substrats kératiniques', on entend selon l'invention une composition destinée à améliorer l'aspect et/ou l'état de surface des substrats kératiniques. Le soin des substrats kératiniques pourra consister à rendre les cheveux plus doux, plus lisses, moins fourchus ; et les ongles moins cassants, moins dédoublés.

Par 'renforcement et/ou réparation des substrats kératiniques' selon l'invention, on entend une composition destinée à conserver et/ou restaurer et/ou améliorer les propriétés physiques et/ou mécaniques des substrats kératiniques, pouvant se manifester par exemple :
- soit par une rigidification des substrats kératiniques, qui leur procure davantage de consistance et de corps, ainsi qu'un toucher plus agréable. Il en résulte par exemple une augmentation du volume des fibres kératiniques ainsi qu'une facilité et un maintien du coiffage plus élevés ;
- soit par une meilleure élasticité et/ou une meilleure résistance à des forces mécaniques de traction qui leur sont appliquées, par exemple lors d'un peignage pour les fibres kératiniques, en particulier sur des cheveux africains ou tous cheveux fragilisés ou sensibilisés.
   Les cheveux sont ainsi plus forts, plus souples, plus doux, plus lisses et plus faciles à coiffer. Les ongles sont ainsi plus lisses, plus brillants, moins abîmés, moins dédoublés et moins cassants.

Selon un premier mode, la composition pourra être appliquée en pré-traitement d'un traitement susceptible de sensibiliser les substrats kératiniques (ex : avant une coloration d'oxydation sur les cheveux).

Selon un autre mode de réalisation, la composition pourra ère appliquée en post-traitement d'un traitement susceptible de sensibiliser les substrats kératiniques (ex : après un traitement de peeling sur la peau).

La composition contenant le polypeptide de la famille des KAP pourra également constituer la composition de traitement (ex : associé à un colorant).

L'application des compositions selon le procédé de l'invention se fera selon la technique d'utilisation habituelle desdites compositions. Par exemple :
- l'application sur cheveux peut avoir lieu avant (lotion, 1 heure avant), pendant (shampooing) ou après (lotions sprays) le shampooing ; la composition peut être appliquée sur cheveux secs (laques, sprays, lotions) ou sur cheveux humides (compositions de déformation permanente ou de mise en plis) ;
- l'application d'une base de soin sur les ongles pourra se faire avant ou après la pose du vernis ;
- l'application d'une composition de soin pour cils pourra être appliquée juste après application du mascara;
- l'application d'une composition de soin pour la peau pourra être appliquée juste après une solution de nettoyage.

Les compositions peuvent être appliquées quotidiennement ou à une fréquence de 2 à 3 applications par semaine, ceci pendant une durée de 1 à 3 mois, renouvelable selon le degré d'altération des substrats kératiniques de l'individu à traiter.

En particulier, le procédé selon l'invention sera destiné au soin, au traitement, au renforcement et/ou à la réparation des substrats kératiniques sensibilisées, et préférentiellement des cheveux sensibilisés.

Par 'cheveux sensibilisés', on entend des cheveux fragilisés ou abîmés par des traitements capillaires comme des défrisants, des permanentes ou des colorations, ou par l'effet d'agents atmosphériques. Les cheveux sensibilisés sont rêches après le lavage et le séchage, avec une moindre tenue mécanique, davantage d'électricité statique et un aspect terne.

L'invention concerne encore l'utilisation cosmétique d'au moins un polypeptide de la famille des KAP dans une composition, comme agent destiné à maintenir et/ou restaurer et/ou améliorer le contenu protéique du substrat kératinique.

En particulier, ledit polypeptide de la famille des KAP sera notamment destiné à :
- maintenir et/ou restaurer et/ou améliorer le contenu protéique du substrat kératinique ;
- maintenir et/ou restaurer et/ou améliorer l'état de surface desdites substrats kératiniques ;
- conserver et/ou restaurer et/ou améliorer les propriétés physiques et/ou mécaniques des substrats kératiniques.

Ledit agent sera ainsi notamment destiné à améliorer la douceur des fibres kératiniques, et/ou leur souplesse et/ou leur élasticité et/ou leur résistance à la casse, et/ou leur volume et/ou leur maintien à la coiffure, et/ou leur résistance aux traitements chimiques.

Les compositions selon l'invention seront ainsi destinées à améliorer l'aspect et/ou l'état de surface des substrats kératiniques, en particulier à rendre les cheveux plus doux, plus lisses, moins fourchus, plus résistants à la casse, à leur donner plus de consistance et de corps, notamment plus de volume et plus de maintien pour la coiffure, plus d'élasticité et de souplesse, ainsi qu'une meilleure résistance aux traitements susceptibles de les fragiliser (ex : coloration, permanente).

L'invention est illustrée plus en détail dans les exemples non limitatifs suivants.

### EXEMPLES

Les valeurs sont exprimées en % par rapport au poids total de la composition, sauf indication contraire.
Ces formulations sont préparées selon les méthodes usuelles.

### Shampooing

- fragment SPCCR de la SEQ ID N°9 1 %
- lauryl ether sulfate de sodium 15%
- eau purifiée qsp 100%

### Mascara

- fragment SPCCR de la SEQ ID N°9 1g
- cire de carnauba 20g
- Stéarate de glycéryle polyoxyéthyléné (30 OE) (Tagat S de la société GOLDSCHMIDT) 8g
- Oxyde de fer noir 5g
- Propylène glycol 5g
- Hydroxyéthylcellulose 2.5g
- Eau qsp 100g

## Revendications

1. Composition cosmétique comprenant, dans un milieu physiologiquement acceptable adapté à une application topique sur les substrats kératiniques, au moins un polypeptide de la famille des KAP et au moins un ingrédient cosmétique choisi parmi les agents de conditionnement, les agents de soin et/ou de traitement des substrats kératiniques et leurs mélanges.

2. Composition selon la revendication 1, **caractérisée en ce que** les agents de conditionnement des fibres kératiniques sont choisis parmi les huiles, les cires, les céramides, les gommes, les silicones, les polysiloxanes, les polymères filmogènes, les polymères fixants et leurs mélanges.

3. Composition selon la revendication 1, **caractérisée en ce que** les agents de soin et/ou de traitement des substrats kératiniques sont choisis parmi les agents hydratants, les agents stimulant la synthèse de macromolécules dermiques ou épidermiques et/ou empêchant leur dégradation, les agents adoucissants, les agents anti-radicalaires, les vitamines, les acides aminés, les protéines, les agents anti-pelliculaires, les agents anti-séborrhéiques, les agents réducteurs, les précurseurs de colorants capillaires, les colorants capillaires directs, les agents oxydants et leurs mélanges.

4. Composition selon l'une quelconque des revendications 1 à 3, **caractérisée en ce que** le(s) ingrédient(s) cosmétique(s) est présent dans la composition en une quantité allant de 0,001 à 20% en poids par rapport au poids total de la composition, de préférence de 0,01 à 10% et encore plus préférentiellement de 0,1 à 2%.

5. Composition selon l'une quelconque des revendications 1 à 4, **caractérisée en ce qu'**elle comprend en outre au moins un adjuvant cosmétique ou agent de formulation choisi parmi les solvants organiques, les agents tensioactifs, les agents plastifiants, les agents épaississants, les agents émulsionnants, les conservateurs, les filtres UV, les charges, les matières colorantes, les parfums, les agents opacifiants, les agents humectants, les agents d'ajustement et de fixation du pH, les agents anti-bactériens, les agents antifongiques, les agents propulseurs et leurs mélanges.

6. Composition selon l'une quelconque des revendications 1 à 5, **caractérisée en ce que** le polypeptide de la famille des KAP possède une séquence peptidique choisie parmi (i) les séquences SEQ ID N° 1 à SEQ ID N°66, (ii) un fragment desdites séquences comprenant au moins 3 acides aminés consécutifs d'une séquence répétée choisie parmi la séquence SZCCXPSCCXP (Z : ∅, P et X : Q,V,R,I) et la séquence YGGXGYGSGY (X:Y,L,F) et (iii) leurs homologues.

7. Composition selon la revendication 6, **caractérisée en ce que** ledit fragment peptidique (ii) contient de 3 à 60 acides aminés, en particulier de 3 à 20 acides aminés et encore plus préférentiellement de 3 à 10 acides aminés.

8. Composition selon l'une quelconque des revendications 1 à 7, **caractérisée en ce que** ledit polypeptide de la famille des KAP ou fragment peptidique de celui-ci est d'origine naturelle ou synthétique.

9. Composition selon l'une quelconque des revendications 1 à 8, **caractérisée en ce que** ledit polypeptide de la famille des KAP est présent dans la composition en une quantité allant de 0,001 à 30% en poids par rapport au poids total de la composition, de préférence de 0,01 à 15% et encore plus préférentiellement de 0,1 à 5%.

10. Composition selon l'une quelconque des revendications 1 à 9, **caractérisée en ce qu'**elle est sous forme de solution aqueuse, hydroalcoolique ou huileuse, d'émulsions huile-dans-eau (H/E) ou eau-dans-huile (E/H) ou multiple (E/H/E ou H/E/H), de gel aqueux ou huileux, de produits anhydres déshydratés, ou de dispersions d'une phase huileuse dans une phase aqueuse à l'aide de sphérules de type nanoparticules ou vésicules lipidiques de type ionique et/ou non ionique.

11. Composition selon l'une quelconque des revendications 1 à 10, **caractérisée en ce qu'**il s'agit d'une lotion de soin capillaire, d'un shampooing ou d'un après-shampooing capillaire, d'un savon liquide ou solide de nettoyage, d'un produit de mise en forme de la coiffure, d'un shampoing colorant, d'une teinture, d'une composition de permanente, d'un masque traitant, d'une crème, d'un gel moussant de nettoyage, d'un mascara de maquillage des cils, d'une base de maquillage des cils, d'une composition top-coat à appliquer sur le mascara, d'une composition de traitement cosmétique des cils, d'un vernis à ongles, d'une base pour les ongles ou "base-coat", d'une composition de finition ou "top-coat", d'un dissolvant pour vernis à ongles, d'une base traitante pour les ongles, d'une crème, d'une pommade, d'un lait, d'une lotion, d'un sérum, d'une pâte, d'une mousse, ou d'un stick.

12. Procédé cosmétique de soin, de traitement, de renforcement et/ou de réparation des substrats kératiniques, **caractérisé en ce que** l'on applique sur la peau, les cheveux, les cils ou les ongles une composition telle que définie dans l'une des revendications 1 à 11, suivi éventuellement d'un rinçage.

13. Procédé cosmétique selon la revendication 12, **caractérisé en ce que** la composition est appliquée en pré-traitement, lors d'un traitement susceptible de sensibiliser les substrats kératiniques et/ou en post-traitement.

14. Procédé cosmétique selon l'une des revendications 12 ou 13, **caractérisé en ce que** la composition est appliquée sur des substrats kératiniques sensibilisés, en particulier des fibres kératiniques sensibilisées.

15. Utilisation cosmétique d'au moins un polypeptide de la famille des KAP dans une composition, comme agent destiné à maintenir et/ou restaurer et/ou améliorer le contenu protéique du substrat kératinique.

16. Utilisation selon la revendication 15, **caractérisée en ce que** le polypeptide de la famille des KAP est destiné à conserver et/ou restaurer et/ou améliorer les propriétés physiques et/ou mécaniques des substrats kératiniques.

17. Utilisation selon la revendication 15, **caractérisée en ce que** le polypeptide de la famille des KAP est destiné à maintenir et/ou restaurer et/ou améliorer l'état de surface desdites substrats kératiniques.

18. Utilisation selon l'une quelconque des revendications 15 à 17, **caractérisée en ce que** ledit agent est destiné à améliorer la douceur des fibres kératiniques, et/ou leur souplesse et/ou leur élasticité et/ou leur résistance à la casse, et/ou leur volume et/ou leur maintien à la coiffure, et/ou leur résistance aux traitements chimiques.


### Annexe aux documents de la demande - listage des séquences déposé ultérieurement

SEQUENCE LISTING

<110>  L'OREAL

<120>  COMPOSITIONS COSMETIQUES DE SOIN, RENFORCEMENT ET/OU REPARATION
       DES SUBSTRATS KERATINIQUES COMPRENANT DES POLYPEPTIDES KAP

<130>  OA05076/CB

<140>  06290415.6
<141>  2006-03-14

<160>  66

<170>  PatentIn version 3.3

<210>  1
<211>  177
<212>  PRT
<213>  Homo sapiens

<400>  1

Met Ala Cys Cys Gln Thr Ser Phe Cys Gly Phe Pro Ser Cys Ser Thr
1               5                   10                  15

Ser Gly Thr Cys Gly Ser Ser Cys Cys Gln Pro Ser Cys Cys Glu Thr
            20                  25                  30

Ser Ser Cys Gln Pro Arg Cys Cys Glu Thr Ser Cys Cys Gln Pro Ser
        35                  40                  45

Cys Cys Gln Thr Ser Phe Cys Gly Phe Pro Ser Phe Ser Thr Gly Gly
    50                  55                  60

Thr Cys Asp Ser Ser Cys Cys Gln Pro Ser Cys Cys Glu Thr Ser Cys
65                  70                  75                  80

Cys Gln Pro Ser Cys Tyr Gln Thr Ser Ser Cys Gly Thr Gly Cys Gly
                85                  90                  95

Ile Gly Gly Gly Ile Gly Tyr Gly Gln Glu Gly Ser Ser Gly Ala Val
            100                 105                 110

Ser Thr Arg Ile Arg Trp Cys Arg Pro Asp Cys Arg Val Glu Gly Thr
        115                 120                 125

Cys Leu Pro Pro Cys Cys Val Val Ser Cys Thr Pro Pro Ser Cys Cys
    130                 135                 140

Gln Leu His His Ala Glu Ala Ser Cys Cys Arg Pro Ser Tyr Cys Gly
145                 150                 155                 160

Gln Ser Cys Cys Arg Pro Val Cys Cys Cys Tyr Cys Ser Glu Pro Thr
                165                 170                 175

Cys

<210>  2
<211>  167
<212>  PRT
<213>  Homo sapiens

<400>  2

Met Thr Cys Cys Gln Thr Ser Phe Cys Gly Tyr Pro Ser Cys Ser Thr
1               5                   10                  15

Ser Gly Thr Cys Gly Ser Ser Cys Cys Gln Pro Ser Cys Cys Glu Thr
            20                  25                  30

Ser Cys Cys Gln Pro Ser Cys Cys Gln Thr Ser Phe Cys Gly Phe Pro
        35                  40                  45

Ser Phe Ser Thr Ser Gly Thr Cys Ser Ser Ser Cys Cys Gln Pro Ser
    50                  55                  60

Cys Cys Glu Thr Ser Cys Cys Gln Pro Ser Cys Cys Gln Thr Ser Ser
65                  70                  75                  80

Cys Gly Thr Gly Cys Gly Ile Gly Gly Gly Ile Gly Tyr Gly Gln Glu
                85                  90                  95

Gly Ser Ser Gly Ala Val Ser Thr Arg Ile Arg Trp Cys Arg Pro Asp
            100                 105                 110

Cys Arg Val Glu Gly Thr Cys Leu Pro Pro Cys Cys Val Val Ser Cys
        115                 120                 125

Thr Pro Pro Thr Cys Cys Gln Leu His His Ala Glu Ala Ser Cys Cys
    130                 135                 140

Arg Pro Ser Tyr Cys Gly Gln Ser Cys Cys Arg Pro Val Cys Cys Cys
145                 150                 155                 160

Tyr Ser Cys Glu Pro Thr Cys
                165


<210>  3
<211>  174
<212>  PRT
<213>  Homo sapiens

<400>  3

Met Thr Cys Cys Gln Thr Ser Phe Cys Gly Tyr Pro Ser Phe Ser Ile
1               5                   10                  15

Ser Gly Thr Cys Gly Ser Ser Cys Cys Gln Pro Ser Cys Cys Glu Thr
            20                  25                  30

Ser Cys Cys Gln Pro Arg Ser Cys Gln Thr Ser Phe Cys Gly Phe Pro
        35                  40                  45

Ser Phe Ser Thr Ser Gly Thr Cys Ser Ser Ser Cys Cys Gln Pro Ser
    50                  55                  60

Cys Cys Glu Thr Ser Cys Cys Gln Pro Ser Cys Cys Glu Thr Ser Cys
65                  70                  75                  80

Cys Gln Pro Ser Cys Cys Gln Ile Ser Ser Cys Gly Thr Gly Cys Gly
                85                  90                  95

Ile Gly Gly Gly Ile Ser Tyr Gly Gln Glu Gly Ser Ser Gly Ala Val
            100                 105                 110

Ser Thr Arg Ile Arg Trp Cys Arg Pro Asp Ser Arg Val Glu Gly Thr
        115                 120                 125

Tyr Leu Pro Pro Cys Cys Val Val Ser Cys Thr Pro Pro Ser Cys Cys
    130                 135                 140

Gln Leu His His Ala Gln Ala Ser Cys Cys Arg Pro Ser Tyr Cys Gly
145                 150                 155                 160

Gln Ser Cys Cys Arg Pro Val Cys Cys Cys Glu Pro Thr Cys
                165                 170

<210>  4
<211>  131
<212>  PRT
<213>  Homo sapiens

<400>  4

Met Ala Cys Cys Gln Thr Ser Phe Cys Gly Phe Arg Ser Phe Ser Thr
1               5                   10                  15

Gly Gly Thr Cys Asp Ser Ser Cys Cys Gln Pro Ser Cys Cys Glu Thr
            20                  25                  30

Ser Cys Cys Gln Pro Ser Cys Tyr Gln Thr Ser Ser Cys Gly Thr Gly
        35                  40                  45

Cys Gly Ile Gly Gly Gly Ile Gly Tyr Gly Gln Glu Gly Ser Ser Gly
    50                  55                  60

Ala Val Ser Thr Arg Ile Arg Trp Cys Arg Pro Asp Cys Arg Val Glu
65                  70                  75                  80

Gly Thr Cys Leu Pro Pro Cys Cys Val Val Ser Cys Thr Pro Pro Ser
                85                  90                  95

Cys Cys Gln Leu His His Ala Glu Ala Ser Cys Cys Arg Pro Ser Tyr
            100                 105                 110

Cys Gly Gln Ser Cys Cys Arg Pro Val Cys Cys Cys Tyr Cys Ser Glu
        115                 120                 125

Pro Thr Cys
    130

<210>  5
<211>  85
<212>  PRT
<213>  Homo sapiens

<400>  5

Met Ala Cys Cys Gln Thr Ser Phe Cys Gly Phe Pro Ser Cys Ser Thr
1               5                   10                  15

Ser Gly Ala Val Ser Thr Arg Ile Arg Trp Cys Arg Pro Asp Cys Arg
            20                  25                  30

Val Glu Gly Thr Cys Leu Pro Pro Cys Cys Val Val Ser Cys Thr Pro
        35                  40                  45

Pro Ser Cys Cys Gln Leu His His Ala Glu Ala Ser Cys Cys Arg Pro
    50                  55                  60

Ser Tyr Cys Gly Gln Ser Cys Cys Arg Pro Val Cys Cys Cys Tyr Cys
65                  70                  75                  80

Ser Glu Pro Thr Cys
                85

<210>  6
<211>  177
<212>  PRT
<213>  Homo sapiens

<400>  6

<210>  7
<211>  141
<212>  PRT
<213>  Homo sapiens

<400>  7

Met Thr Cys Cys Gln Thr Ser Phe Cys Gly Tyr Pro Ser Cys Ser Thr
1               5                   10                  15

Ser Gly Thr Cys Gly Ser Ser Cys Cys Gln Pro Ser Cys Cys Glu Thr
            20                  25                  30

Ser Cys Cys Gln Pro Ser Cys Cys Glu Thr Ser Cys Cys Gln Pro Ser
        35                  40                  45

Cys Cys Gln Thr Ser Ser Cys Gly Thr Gly Cys Gly Ile Gly Gly Gly
    50                  55                  60

Ile Gly Tyr Gly Gln Glu Gly Ser Ser Gly Ala Val Ser Thr Arg Ile
65                  70                  75                  80

Arg Trp Cys Arg Pro Asp Cys Arg Val Glu Gly Thr Cys Leu Pro Pro
                85                  90                  95

Cys Cys Val Val Ser Cys Thr Pro Pro Thr Cys Cys Gln Leu His His
            100                 105                 110

Ala Glu Ala Ser Cys Cys Arg Pro Ser Tyr Cys Gly Gln Ser Cys Cys
        115                 120                 125

Arg Pro Val Cys Cys Cys Tyr Ser Cys Glu Pro Thr Cys
    130                 135                 140

<210>  8
<211>  96
<212>  PRT
<213>  Homo sapiens

<400>  8

Pro Val Thr Cys Gln Thr Thr Val Cys Arg Pro Val Thr Cys Val Pro
1               5                   10                  15

Arg Cys Thr Arg Pro Ile Cys Glu Pro Cys Arg Arg Pro Val Cys Cys
            20                  25                  30

Asp Pro Cys Ser Leu Gln Glu Gly Cys Cys Arg Pro Ile Thr Cys Cys
        35                  40                  45

Pro Ser Ser Cys Thr Ala Val Val Cys Arg Pro Cys Cys Trp Ala Thr
    50                  55                  60

Thr Cys Cys Gln Pro Val Ser Val Gln Ser Pro Cys Cys Arg Pro Pro
65                  70                  75                  80

Cys Gly Gln Pro Thr Pro Cys Ser Thr Thr Cys Arg Thr Ser Ser Cys
                85                  90                  95

<210>  9
<211>  33
<212>  PRT
<213>  Homo sapiens

<400>  9

Thr Thr Cys Cys Gln Pro Val Ser Val Gln Ser Pro Cys Cys Arg Pro
1               5                   10                  15

Pro Cys Gly Gln Pro Thr Pro Cys Ser Thr Thr Cys Arg Thr Ser Ser
            20                  25                  30

Cys

<210>  10
<211>  85
<212>  PRT
<213>  Homo sapiens

<400>  10

Val Thr Cys Val Pro Arg Cys Thr Arg Pro Ile Cys Glu Pro Cys Arg
1               5                   10                  15

Arg Pro Val Cys Cys Asp Pro Cys Ser Leu Gln Glu Gly Cys Cys Arg
            20                  25                  30

Pro Ile Thr Cys Cys Pro Ser Ser Cys Thr Ala Val Val Cys Arg Pro
        35                  40                  45

Cys Cys Trp Ala Thr Thr Cys Cys Gln Pro Val Ser Val Gln Ser Pro
    50                  55                  60

Cys Cys Arg Pro Pro Cys Gly Gln Pro Thr Pro Cys Ser Thr Thr Cys
65                  70                  75                  80

Arg Thr Ser Ser Cys
                85

<210>  11
<211>  128
<212>  PRT
<213>  Homo sapiens

<400>  11

Met Thr Gly Ser Cys Cys Gly Ser Thr Leu Ser Ser Leu Ser Tyr Gly
1               5                   10                  15

Gly Gly Cys Cys Gln Pro Cys Cys Cys Arg Asp Pro Cys Cys Cys Arg
            20                  25                  30

Pro Val Thr Cys Gln Thr Thr Val Cys Arg Pro Val Thr Cys Val Pro
        35                  40                  45

Arg Cys Thr Arg Pro Ile Cys Glu Pro Cys Arg Arg Pro Val Cys Cys
    50                  55                  60

Asp Pro Cys Ser Leu Gln Glu Gly Cys Cys Arg Pro Ile Thr Cys Cys
65                  70                  75                  80

Pro Ser Ser Cys Thr Ala Val Val Cys Arg Pro Cys Cys Trp Ala Thr
                85                  90                  95

Thr Cys Cys Gln Pro Val Ser Val Gln Ser Pro Cys Cys Arg Pro Pro
            100                 105                 110

Cys Gly Gln Pro Thr Pro Cys Ser Thr Thr Cys Arg Thr Ser Ser Cys
        115                 120                 125

<210>  12
<211>  98
<212>  PRT
<213>  Homo sapiens

<400>  12

Met Tyr Cys Cys Ala Leu Arg Ser Cys Ser Val Pro Thr Gly Pro Ala
1               5                   10                  15

Thr Thr Phe Cys Ser Phe Asp Lys Ser Cys Arg Cys Gly Val Cys Leu
            20                  25                  30

Pro Ser Thr Cys Pro His Glu Ile Ser Leu Leu Gln Pro Ile Cys Cys
        35                  40                  45

Asp Thr Cys Pro Pro Pro Cys Cys Lys Pro Asp Thr Tyr Val Pro Thr
    50                  55                  60

Cys Trp Leu Leu Asn Asn Cys His Pro Thr Pro Gly Leu Ser Gly Ile
65                  70                  75                  80

Asn Leu Thr Thr Tyr Val Gln Pro Gly Cys Glu Ser Pro Cys Glu Pro
                85                  90                  95

Arg Cys

<210>  13
<211>  98
<212>  PRT
<213>  Homo sapiens

<400>  13

Met Asp Cys Cys Ala Ser Arg Ser Cys Ser Val Pro Thr Gly Pro Ala
1               5                   10                  15

Thr Thr Ile Cys Ser Ser Asp Lys Ser Cys Arg Cys Gly Val Cys Leu
            20                  25                  30

Pro Ser Thr Cys Pro His Thr Val Trp Leu Leu Glu Pro Ile Cys Cys
        35                  40                  45

Asp Asn Cys Pro Pro Pro Cys His Ile Pro Gln Pro Cys Val Pro Thr
    50                  55                  60

Cys Phe Leu Leu Asn Ser Cys Gln Pro Thr Pro Gly Leu Glu Thr Leu
65                  70                  75                  80

Asn Leu Thr Thr Phe Thr Gln Pro Cys Cys Glu Pro Cys Leu Pro Arg
                85                  90                  95

Gly Cys

<210>  14
<211>  98
<212>  PRT
<213>  Homo sapiens

<400>  14

Met Asp Cys Cys Ala Ser Arg Gly Cys Ser Val Pro Thr Gly Pro Ala
1               5                   10                  15

Thr Thr Ile Cys Ser Ser Asp Lys Ser Cys Arg Cys Gly Val Cys Leu
            20                  25                  30

Pro Ser Thr Cys Pro His Thr Val Trp Leu Leu Glu Pro Thr Cys Cys
        35                  40                  45

Asp Asn Cys Pro Pro Pro Cys His Ile Pro Gln Pro Cys Val Pro Thr
    50                  55                  60

Cys Phe Leu Leu Asn Ser Cys Gln Pro Thr Pro Gly Leu Glu Thr Leu
65                  70                  75                  80

Asn Leu Thr Thr Phe Thr Gln Pro Cys Cys Glu Pro Cys Leu Pro Arg
                85                  90                  95

Gly Cys

<210>  15
<211>  136
<212>  PRT
<213>  Homo sapiens

<400>  15

Met Val Asn Ser Cys Cys Gly Ser Val Cys Ser Asp Gln Gly Cys Gly
1               5                   10                  15

Leu Glu Asn Cys Cys Arg Pro Ser Cys Cys Gln Thr Thr Cys Cys Arg
            20                  25                  30

Thr Thr Cys Cys Arg Pro Ser Cys Cys Val Ser Ser Cys Cys Arg Pro
        35                  40                  45

Gln Cys Cys Gln Ser Val Cys Cys Gln Pro Thr Cys Cys Ser Pro Ser
    50                  55                  60

Cys Cys Gln Thr Thr Cys Cys Arg Thr Thr Cys Cys Arg Pro Ser Cys
65                  70                  75                  80

Cys Val Ser Ser Cys Phe Arg Pro Gln Cys Cys Gln Ser Val Cys Cys
                85                  90                  95

Gln Pro Thr Cys Cys Arg Pro Ser Cys Gly Gln Thr Thr Cys Cys Arg
            100                 105                 110

Thr Thr Cys Tyr Arg Pro Ser Cys Cys Val Ser Thr Cys Cys Arg Pro
        115                 120                 125

Thr Cys Ser Ser Gly Ser Cys Cys
    130                 135

<210>  16
<211>  98
<212>  PRT
<213>  Homo sapiens

<400>  16

Thr Cys Cys Arg Pro Ser Cys Cys Ile Ser Ser Cys Cys Arg Pro Ser
1               5                   10                  15

Cys Cys Ile Ser Ser Cys Cys Lys Pro Ser Cys Cys Gln Thr Thr Cys
            20                  25                  30

Cys Arg Pro Ser Cys Cys Ile Ser Ser Cys Tyr Arg Pro Gln Cys Cys
        35                  40                  45

Gln Pro Ser Cys Cys Arg Pro Ala Cys Cys Ile Ser Ser Cys Cys His
    50                  55                  60

Pro Ser Cys Cys Val Ser Ser Cys Arg Cys Pro Phe Ser Cys Pro Thr
65                  70                  75                  80

Thr Cys Cys Arg Thr Thr Cys Phe His Pro Ile Cys Cys Gly Ser Ser
                85                  90                  95

Cys Cys

<210>  17
<211>  166
<212>  PRT
<213>  Homo sapiens

<400>  17

Met Val Asn Ser Cys Cys Gly Ser Val Cys Ser Asp Gln Gly Cys Gly
1               5                   10                  15

Leu Glu Asn Cys Cys Arg Pro Ser Tyr Cys Gln Thr Thr Cys Cys Arg
            20                  25                  30

Thr Thr Cys Cys Arg Pro Ser Cys Cys Val Ser Ser Cys Cys Arg Pro
        35                  40                  45

Gln Cys Cys Gln Thr Thr Cys Cys Arg Thr Thr Cys Cys His Pro Ser
    50                  55                  60

Cys Cys Val Ser Ser Cys Cys Arg Pro Gln Cys Cys Gln Ser Val Cys
65                  70                  75                  80

Cys Gln Pro Thr Cys Cys Arg Pro Gln Cys Cys Gln Thr Thr Cys Cys
                85                  90                  95

Arg Thr Thr Cys Cys Arg Pro Ser Cys Cys Arg Pro Gln Cys Cys Gln

            100                 105                 110

Ser Val Cys Cys Gln Pro Thr Cys Cys Cys Pro Ser Tyr Cys Val Ser
        115                 120                 125

Ser Cys Cys Arg Pro Gln Cys Cys Gln Thr Thr Cys Cys Arg Thr Thr
    130                 135                 140

Cys Cys Arg Pro Ser Cys Cys Val Ser Arg Cys Tyr Arg Pro His Cys
145                 150                 155                 160

Gly Gln Ser Leu Cys Cys
                165

<210> 18
<211> 186
<212> PRT
<213> Homo sapiens

<400> 18

Met Val Ser Ser Cys Cys Gly Ser Val Ser Ser Glu Gln Ser Cys Gly
1               5                   10                  15

Leu Glu Asn Cys Cys Cys Pro Ser Cys Cys Gln Thr Thr Cys Cys Arg
            20                  25                  30

Thr Thr Cys Cys Arg Pro Ser Cys Cys Lys Pro Gln Cys Cys Gln Ser
        35                  40                  45

Val Cys Tyr Gln Pro Thr Cys Cys His Pro Ser Cys Cys Ile Ser Ser
    50                  55                  60

Cys Cys Arg Pro Tyr Cys Cys Glu Ser Ser Cys Cys Arg Pro Cys Cys
65                  70                  75                  80

Cys Arg Pro Ser Cys Cys Gln Thr Thr Cys Cys Arg Thr Thr Cys Cys
                85                  90                  95

Arg Thr Thr Cys Cys Cys Pro Ser Cys Cys Val Ser Ser Cys Cys Arg
            100                 105                 110

Pro Gln Cys Cys Gln Ser Val Cys Cys Gln Pro Thr Cys Cys Arg Pro
        115                 120                 125

Ser Tyr Cys Ile Ser Ser Cys Cys His Pro Ser Cys Cys Glu Ser Ser
    130                 135                 140

Cys Cys Arg Pro Cys Cys Cys Val Arg Pro Val Cys Gly Arg Val Ser
145                 150                 155                 160

Cys His Thr Thr Cys Tyr Arg Pro Thr Cys Val Ile Ser Thr Cys Pro
                165                 170                 175

Arg Pro Leu Cys Cys Ala Ser Ser Cys Cys
            180                 185

<210>  19
<211>  109
<212>  PRT
<213>  Homo sapiens

<400>  19

Ser Pro Leu Ala Ala Val Pro Ala Ala Ala Ser Pro Ala Ala Val Ala
1               5                   10                  15

Pro Ala Ala Val Cys Pro Gly Ala Ala Gly Pro Ser Ala Ala Ser Leu
            20                  25                  30

Cys Ala Ala Ser Gln Pro Ala Ala Val Pro Ala Ala Ala Ser Pro Ala
        35                  40                  45

Ala Ala Ala Pro Leu Ala Val Asn Pro Ala Ala Ala Ala Arg Ala Ala
    50                  55                  60

Ala Ala Pro Ala Ala Ala Cys Val Gln Ser Val Ala Glu Ser Pro Ala
65                  70                  75                  80

Thr Pro Leu Ala Ile Ala Gln Pro Val Ser Phe Pro Pro Ala Pro Val
                85                  90                  95

Pro Cys Ala Val Pro Pro Leu Ala Ala Glu Leu Leu Ala
            100                 105

<210>  20
<211>  210
<212>  PRT
<213>  Homo sapiens

<400>  20

Met Val Ser Ser Cys Cys Gly Ser Val Cys Ser Asp Gln Gly Cys Gly
1               5                   10                  15

Gln Val Leu Cys Gln Glu Thr Cys Cys Arg Pro Ser Cys Cys Gln Thr
            20                  25                  30

Thr Cys Cys Arg Thr Thr Cys Tyr Arg Pro Ser Cys Cys Val Ser Ser
        35                  40                  45

Cys Cys Arg Pro Gln Cys Cys Gln Ser Val Cys Cys Gln Pro Thr Cys
    50                  55                  60

Cys Arg Pro Ser Cys Cys Glu Thr Thr Cys Cys His Pro Arg Cys Cys
65                  70                  75                  80

Ile Ser Ser Cys Cys Arg Pro Ser Cys Cys Met Ser Ser Cys Cys Lys
                85                  90                  95

Pro Gln Cys Cys Gln Ser Val Cys Cys Gln Pro Thr Cys Cys His Pro
            100                 105                 110

Ser Cys Cys Ile Ser Ser Cys Cys Arg Pro Ser Cys Cys Val Ser Arg
        115                 120                 125

Cys Cys Arg Pro Gln Cys Cys Gln Ser Val Cys Cys Gln Pro Thr Cys
    130                 135                 140

Cys Arg Pro Ser Cys Cys Ile Ser Ser Cys Cys Arg Pro Ser Cys Cys
145                 150                 155                 160

Glu Ser Ser Cys Cys Arg Pro Cys Cys Cys Arg Pro Cys Cys Cys Leu
                165                 170                 175

Arg Pro Val Cys Gly Arg Val Ser Cys His Thr Thr Cys Tyr Arg Pro
            180                 185                 190

Thr Cys Val Ile Ser Thr Cys Pro Arg Pro Leu Cys Cys Ala Ser Ser
        195                 200                 205

Cys Cys
    210

<210>  21
<211>  114
<212>  PRT
<213>  Homo sapiens

<400>  21

Ser Ser Cys Cys Lys Pro Gln Cys Cys Gln Ser Val Cys Cys Gln Pro
1               5                   10                  15

Thr Cys Cys His Pro Ser Cys Cys Ile Ser Ser Cys Cys Arg Pro Ser
            20                  25                  30

Cys Cys Val Ser Ser Cys Cys Lys Pro Gln Cys Cys Gln Ser Val Cys
        35                  40                  45

Cys Gln Pro Thr Cys Cys His Pro Ser Cys Ser Ile Ser Ser Cys Cys
    50                  55                  60

Arg Pro Ser Cys Cys Glu Ser Ser Cys Cys Arg Pro Cys Cys Cys Val
65                  70                  75                  80

Arg Pro Val Cys Gly Arg Val Ser Cys His Thr Thr Cys Tyr Arg Pro
                85                  90                  95

Thr Cys Val Ile Ser Thr Cys Pro Arg Pro Val Cys Cys Ala Ser Ser

100                 105                 110

Cys Cys

<210>  22
<211>  191
<212>  PRT
<213>  Homo sapiens

<400>  22

Cys Gln Glu Thr Cys Cys Arg Pro Ser Cys Cys Glu Thr Thr Cys Cys
1               5                   10                  15

Arg Thr Thr Cys Cys Arg Pro Ser Cys Cys Val Ser Ser Cys Cys Arg
            20                  25                  30

Pro Gln Cys Cys Gln Ser Val Cys Cys Gln Pro Thr Cys Ser Arg Pro
        35                  40                  45

Ser Cys Cys Gln Thr Thr Cys Cys Arg Thr Thr Cys Tyr Arg Pro Ser
    50                  55                  60

Cys Cys Val Ser Ser Cys Cys Arg Pro Gln Cys Cys Gln Pro Val Cys
65                  70                  75                  80

Cys Gln Pro Thr Cys Cys Arg Pro Ser Cys Cys Glu Thr Thr Cys Cys
                85                  90                  95

His Pro Arg Cys Cys Ile Ser Ser Cys Cys Arg Pro Ser Cys Cys Val
            100                 105                 110

Ser Ser Cys Cys Lys Pro Gln Cys Cys Gln Ser Val Cys Cys Gln Pro
        115                 120                 125

Asn Cys Cys Arg Pro Ser Cys Ser Ile Ser Ser Cys Cys Arg Pro Ser
    130                 135                 140

Cys Cys Glu Ser Ser Cys Cys Arg Pro Cys Cys Cys Val Arg Pro Val
145                 150                 155                 160

Cys Gly Arg Val Ser Cys His Thr Thr Cys Tyr Arg Pro Thr Cys Val
                165                 170                 175

Ile Ser Ser Cys Pro Arg Pro Leu Cys Cys Ala Ser Ser Cys Cys
            180                 185                 190

<210>  23
<211>  127
<212>  PRT
<213>  Homo sapiens

<400>  23

Met Val Asn Ser Cys Cys Gly Ser Val Cys Ser Asp Gln Gly Cys Asp
1               5                   10                  15

Gln Gly Leu Cys Gln Glu Thr Cys Cys Arg Pro Ser Cys Cys Gln Thr
            20                  25                  30

Thr Cys Cys Cys Pro Ser Cys Val Val Ser Ser Cys Cys Arg Pro Ser
        35                  40                  45

Cys Ser Gln Thr Thr Cys Cys Gln Thr Thr Cys Cys Arg Pro Ser Cys
    50                  55                  60

Cys Arg Pro Val Cys Cys Gln Thr Thr Cys Arg Pro Ser Cys Gly Val
65                  70                  75                  80

Ser Ser Cys Cys Arg Pro Leu Cys Cys Gln Thr Thr Cys Arg Pro Ser
                85                  90                  95

Cys Gly Val Ser Ser Cys Cys Arg Pro Leu Cys Cys Gln Thr Thr Cys
            100                 105                 110

Cys Arg Thr Thr Cys Cys Arg Pro Ser Cys Cys Gly Ser Ser Cys
        115                 120                 125

<210>  24
<211>  201
<212>  PRT
<213>  Homo sapiens

<400>  24

Met Val Asn Ser Cys Cys Gly Ser Val Cys Ser Asp Gln Gly Cys Gly
1               5                   10                  15

Leu Glu Asn Cys Cys Arg Pro Ser Cys Cys Gln Thr Thr Cys Cys Arg
            20                  25                  30

Thr Thr Cys Cys Arg Pro Ser Cys Cys Val Ser Ser Cys Cys Arg Pro
        35                  40                  45

Gln Cys Cys Gln Ser Val Cys Cys Gln Pro Thr Cys Cys Arg Pro Ser
    50                  55                  60

Cys Cys Gln Thr Thr Cys Cys Arg Thr Thr Cys Cys Arg Pro Ser Cys
65                  70                  75                  80

Cys Val Ser Ser Cys Cys Arg Pro Gln Cys Cys Gln Ser Val Cys Cys
                85                  90                  95

Gln Pro Thr Cys Cys Arg Pro Ser Cys Cys Gln Thr Thr Cys Cys Arg
            100                 105                 110

Thr Thr Cys Cys Arg Pro Ser Cys Cys Val Ser Ser Cys Cys Arg Pro
        115                 120                 125

Gln Cys Cys Gln Ser Val Cys Cys Gln Pro Thr Cys Cys Arg Pro Ser
    130                 135                 140

Cys Cys Ile Ser Ser Ser Cys Cys Pro Ser Cys Cys Glu Ser Ser Cys
145                 150                 155                 160

Cys Arg Pro Cys Cys Cys Leu Arg Pro Val Cys Gly Arg Val Ser Cys
                165                 170                 175

His Thr Thr Cys Tyr Arg Pro Thr Cys Val Ile Ser Thr Cys Pro Arg
            180                 185                 190

Pro Leu Cys Cys Ala Ser Ser Cys Cys
        195                 200

<210>  25
<211>  106
<212>  PRT
<213>  Homo sapiens

<400>  25

Met Val Asn Ser Cys Cys Gly Ser Val Cys Ser Asp Gln Gly Cys Gly
1               5                   10                  15

Leu Glu Asn Cys Cys Arg Pro Ser Tyr Cys Gln Thr Thr Cys Cys Arg
            20                  25                  30

Thr Thr Cys Cys Arg Pro Ser Cys Cys Arg Pro Ser Cys Cys Arg Pro
        35                  40                  45

Gln Cys Cys Gln Ser Val Cys Cys Gln Pro Thr Cys Cys Cys Pro Ser
    50                  55                  60

Tyr Cys Val Ser Ser Cys Cys Arg Pro Gln Cys Cys Gln Thr Thr Arg
65                  70                  75                  80

Cys Arg Thr Thr Cys Cys Arg Pro Ser Cys Cys Val Ser Arg Cys Tyr
                85                  90                  95

Arg Pro His Cys Gly Gln Ser Leu Cys Cys
            100                 105

<210>  26
<211>  195
<212>  PRT
<213>  Homo sapiens

<400>  26

Met Val Asn Ser Cys Cys Gly Ser Val Cys Ser His Gln Gly Cys Gly
1               5                   10                  15

Gln Asp Leu Cys Gln Glu Thr Cys Cys Arg Pro Ser Cys Cys Glu Thr
            20                  25                  30

Thr Cys Cys Arg Thr Thr Tyr Cys Arg Pro Ser Cys Cys Val Ser Ser
        35                  40                  45

Cys Cys Arg Pro Gln Cys Cys Gln Ser Val Cys Cys Gln Pro Thr Cys
    50                  55                  60

Cys Arg Pro Arg Cys Cys Ile Ser Ser Cys Cys Arg Pro Ser Cys Cys
65                  70                  75                  80

Val Ser Ser Cys Cys Lys Pro Gln Cys Cys Gln Ser Met Cys Cys Gln
                85                  90                  95

Pro Thr Cys Cys Arg Pro Arg Cys Cys Ile Ser Ser Cys Cys Arg Pro
            100                 105                 110

Ser Cys Cys Val Ser Ser Cys Cys Arg Pro Gln Cys Cys Gln Ser Val
        115                 120                 125

Cys Cys Gln Pro Thr Cys Cys His Pro Ser Cys Ser Ile Ser Ser Cys
    130                 135                 140

Cys Arg Pro Ser Cys Cys Glu Ser Ser Cys Cys Arg Pro Cys Cys Cys
145                 150                 155                 160

Leu Arg Pro Val Cys Gly Gly Val Ser Cys His Thr Thr Cys Tyr Arg
                165                 170                 175

Pro Thr Cys Val Ile Ser Ser Cys Pro Arg Pro Leu Cys Cys Ala Ser
            180                 185                 190

Ser Cys Cys
        195

<210>  27
<211>  193
<212>  PRT
<213>  Homo sapiens

<400>  27

Gln Gly Cys Gly Leu Glu Thr Cys Cys Arg Pro Ser Cys Cys Gln Thr
1               5                   10                  15

Thr Cys Cys Arg Thr Thr Cys Cys Arg Pro Ser Cys Cys Val Ser Ser
            20                  25                  30

Cys Cys Arg Pro Gln Cys Cys Gln Ser Val Cys Cys Gln Pro Thr Cys
        35                  40                  45

Cys Arg Pro Ser Cys Cys Pro Ser Cys Cys Gln Thr Thr Cys Cys Arg
    50                  55                  60

Thr Thr Cys Cys Arg Pro Ser Cys Cys Val Ser Ser Cys Cys Arg Pro
65                  70                  75                  80

Gln Cys Cys Gln Ser Val Cys Cys Gln Pro Thr Cys Cys Arg Pro Ser
                85                  90                  95

Cys Ser Ile Ser Ser Cys Cys Arg Pro Ser Cys Cys Val Ser Arg Cys
            100                 105                 110

Cys Arg Ser Gln Cys Cys Gln Ser Val Cys Cys Gln Pro Thr Cys Cys
        115                 120                 125

Arg Pro Ser Cys Cys Ile Ser Ser Cys Cys Arg Pro Ser Cys Cys Glu
    130                 135                 140

Ser Ser Cys Cys Arg Pro Cys Cys Cys Arg Pro Cys Cys Cys Leu Arg
145                 150                 155                 160

Pro Val Cys Gly Arg Val Ser Cys His Thr Thr Cys Tyr Arg Pro Thr
                165                 170                 175

Cys Val Ile Ser Thr Cys Pro Arg Pro Leu Cys Cys Ala Ser Ser Cys
            180                 185                 190

Cys

<210>  28
<211>  278
<212>  PRT
<213>  Homo sapiens

<400>  28

Met Gly Cys Cys Gly Cys Ser Gly Gly Cys Gly Ser Ser Cys Gly Gly
1               5                   10                  15

Cys Gly Ser Gly Cys Gly Gly Cys Gly Ser Gly Cys Gly Gly Cys Gly
            20                  25                  30

Ser Gly Cys Gly Gly Ser Gly Ser Ser Cys Cys Val Pro Val Cys Cys
        35                  40                  45

Cys Lys Pro Val Cys Cys Arg Val Pro Thr Cys Ser Cys Ser Ser Cys
    50                  55                  60

Gly Lys Gly Gly Cys Gly Ser Ser Gly Gly Ser Lys Gly Gly Cys Gly
65                  70                  75                  80

Ser Cys Gly Gly Cys Lys Gly Gly Cys Gly Ser Cys Gly Gly Ser Lys
                85                  90                  95

Gly Gly Cys Gly Ser Cys Gly Gly Ser Lys Gly Gly Cys Gly Ser Cys
            100                 105                 110

Gly Gly Ser Lys Gly Gly Cys Gly Ser Gly Cys Gly Gly Cys Gly Ser
        115                 120                 125

Ser Cys Cys Val Pro Val Cys Cys Cys Lys Pro Met Cys Cys Cys Val
    130                 135                 140

Pro Ala Cys Ser Cys Ser Ser Cys Gly Lys Gly Gly Cys Gly Ser Cys
145                 150                 155                 160

Gly Cys Ser Lys Gly Ala Cys Gly Ser Cys Gly Gly Ser Lys Gly Gly
                165                 170                 175

Cys Gly Ser Cys Gly Gly Cys Lys Gly Gly Cys Gly Ser Cys Gly Gly
            180                 185                 190

Ser Lys Gly Gly Cys Gly Ser Gly Cys Gly Gly Cys Gly Ser Gly Cys
        195                 200                 205

Gly Val Pro Val Cys Cys Cys Ser Cys Ser Ser Cys Gly Ser Cys Ala
    210                 215                 220

Gly Ser Lys Gly Gly Cys Gly Ser Ser Cys Ser Gln Cys Ser Cys Cys
225                 230                 235                 240

Lys Pro Cys Cys Cys Ser Ser Gly Cys Gly Ser Ser Cys Cys Gln Ser
                245                 250                 255

Ser Cys Cys Lys Pro Cys Cys Ser Gln Ser Ser Cys Cys Val Pro Val
            260                 265                 270

Cys Cys Gln Cys Lys Ile
        275

<210>  29
<211>  177
<212>  PRT
<213>  Homo sapiens

<400>  29

Met Gly Cys Cys Gly Cys Ser Arg Gly Cys Gly Ser Gly Cys Gly Gly
1               5                   10                  15

Cys Gly Ser Ser Cys Gly Gly Cys Gly Ser Gly Cys Gly Gly Cys Gly
            20                  25                  30

Ser Gly Arg Gly Gly Cys Gly Ser Gly Cys Gly Gly Cys Ser Ser Ser

35                  40                  45

Cys Gly Gly Cys Gly Ser Arg Cys Tyr Val Pro Val Cys Cys Cys Lys
    50                  55                  60

Pro Val Cys Ser Trp Val Pro Ala Cys Ser Cys Thr Ser Cys Gly Ser
65                  70                  75                  80

Cys Gly Gly Ser Lys Gly Gly Cys Gly Ser Cys Gly Gly Ser Lys Gly
                85                  90                  95

Gly Cys Gly Ser Cys Gly Gly Ser Lys Gly Gly Cys Gly Ser Cys Gly
            100                 105                 110

Cys Ser Gln Ser Ser Cys Cys Lys Pro Cys Cys Cys Ser Ser Gly Cys
        115                 120                 125

Gly Ser Ser Cys Cys Gln Ser Ser Cys Cys Lys Pro Cys Cys Cys Gln
    130                 135                 140

Ser Ser Cys Cys Val Pro Val Cys Cys Gln Ser Ser Cys Cys Lys Pro
145                 150                 155                 160

Cys Cys Cys Gln Ser Asn Cys Cys Val Pro Val Cys Cys Gln Cys Lys
                165                 170                 175

Ile

<210>  30
<211>  238
<212>  PRT
<213>  Homo sapiens

<400>  30

Met Gly Cys Ser Gly Cys Ser Gly Gly Cys Gly Ser Ser Cys Gly Gly
1               5                   10                  15

Cys Gly Ser Ser Cys Gly Gly Cys Gly Ser Gly Tyr Gly Gly Cys Gly
            20                  25                  30

Ser Gly Cys Cys Val Pro Val Cys Cys Cys Lys Pro Val Cys Cys Cys
        35                  40                  45

Val Pro Ala Cys Ser Cys Ser Ser Cys Gly Ser Cys Gly Gly Ser Lys
    50                  55                  60

Gly Val Cys Gly Ser Cys Gly Gly Cys Lys Gly Gly Cys Gly Ser Cys
65                  70                  75                  80

Gly Gly Ser Lys Gly Gly Cys Gly Ser Ser Cys Cys Val Pro Val Cys
                85                  90                  95

Cys Ser Ser Ser Cys Gly Ser Cys Gly Gly Ser Lys Gly Val Cys Gly
            100                 105                 110

Phe Arg Gly Gly Ser Lys Gly Gly Cys Gly Ser Cys Gly Cys Ser Gln
        115                 120                 125

Cys Ser Cys Tyr Lys Pro Cys Cys Cys Ser Ser Gly Cys Gly Ser Ser
    130                 135                 140

Cys Cys Gln Ser Ser Cys Cys Lys Pro Ser Cys Ser Gln Ser Ser Cys
145                 150                 155                 160

Cys Lys Pro Cys Cys Ser Gln Ser Ser Cys Cys Lys Pro Cys Cys Cys
                165                 170                 175

Ser Ser Gly Cys Gly Ser Ser Cys Cys Gln Ser Ser Cys Cys Lys Pro
            180                 185                 190

Cys Cys Ser Gln Ser Ser Cys Cys Lys Pro Cys Cys Cys Ser Ser Gly
        195                 200                 205

Cys Gly Ser Ser Cys Cys Gln Ser Ser Cys Cys Lys Pro Cys Ser Ser
    210                 215                 220

Gln Ser Ser Cys Cys Val Pro Ile Cys Cys Gln Cys Lys Ile
225                 230                 235

<210>  31
<211>  288
<212>  PRT
<213>  Homo sapiens

<400>  31

Met Gly Cys Cys Gly Cys Ser Gly Gly Cys Gly Ser Gly Cys Gly Gly
1               5                   10                  15

Cys Gly Ser Gly Cys Gly Gly Cys Gly Ser Gly Cys Gly Gly Cys Gly
            20                  25                  30

Ser Gly Cys Gly Gly Cys Gly Ser Gly Cys Gly Gly Cys Gly Ser Ser
        35                  40                  45

Cys Cys Val Pro Ile Cys Cys Cys Lys Pro Val Cys Cys Cys Val Pro
    50                  55                  60

Ala Cys Ser Cys Ser Ser Cys Gly Ser Cys Gly Gly Ser Lys Gly Gly
65                  70                  75                  80

Tyr Gly Ser Cys Gly Gly Ser Lys Gly Gly Cys Val Ser Cys Gly Gly
                85                  90                  95

Ser Lys Gly Gly Cys Gly Ser Cys Gly Gly Ser Lys Gly Gly Cys Gly
            100                 105                 110

Ser Cys Gly Gly Ser Lys Gly Gly Cys Gly Ser Cys Gly Gly Ser Lys
        115                 120                 125

Gly Gly Cys Val Ser Cys Gly Gly Ser Lys Gly Gly Cys Gly Ser Cys
    130                 135                 140

Gly Gly Ser Lys Gly Gly Cys Val Ser Cys Gly Gly Ser Lys Gly Gly
145                 150                 155                 160

Cys Gly Ser Cys Gly Gly Ser Lys Gly Gly Cys Gly Ser Cys Gly Gly
                165                 170                 175

Ser Lys Gly Gly Cys Gly Ser Cys Gly Gly Ser Lys Gly Gly Cys Gly
            180                 185                 190

Ser Cys Gly Cys Ser Gln Cys Ser Cys Cys Lys Pro Cys Cys Cys Ser
        195                 200                 205

Ser Gly Cys Gly Ser Ser Cys Cys Gln Ser Ser Cys Cys Lys Pro Cys
    210                 215                 220

Cys Ser Ser Ser Gly Cys Gly Ser Ser Cys Cys Gln Ser Ser Cys Cys
225                 230                 235                 240

Lys Pro Tyr Cys Cys Gln Ser Ser Cys Cys Lys Pro Cys Cys Ser Ser
                245                 250                 255

Ser Gly Cys Gly Ser Ser Cys Cys Gln Ser Ser Cys Cys Asn Pro Cys
            260                 265                 270

Cys Ser Gln Ser Ser Cys Cys Val Pro Val Cys Cys Gln Cys Lys Ile
        275                 280                 285

<210> 32
<211> 221
<212> PRT
<213> Homo sapiens

<400> 32

Met Gly Cys Cys Gly Cys Ser Gly Gly Cys Gly Ser Gly Cys Gly Gly
1               5                   10                  15

Leu Gly Ser Gly Cys Gly Gly Cys Gly Ser Gly Cys Gly Gly Arg Gly
            20                  25                  30

Ser Gly Cys Gly Gly Cys Gly Ser Gly Cys Gly Gly Cys Gly Ser Gly
        35                  40                  45

Cys Gly Gly Cys Gly Ser Gly Cys Gly Gly Cys Gly Gly Cys Gly Ser
    50                  55                  60

Gly Cys Cys Val Pro Val Cys Cys Cys Lys Pro Met Cys Cys Cys Val
65                  70                  75                  80

Pro Ala Cys Ser Cys Ser Ser Cys Gly Lys Gly Gly Cys Gly Ser Cys
                85                  90                  95

Gly Gly Ser Lys Arg Gly Cys Val Ser Cys Gly Val Ser Lys Gly Ala
            100                 105                 110

Cys Gly Ser Cys Gly Gly Ser Lys Gly Gly Cys Gly Ser Cys Gly Gly
        115                 120                 125

Ser Lys Gly Gly Cys Gly Ser Cys Gly Gly Ser Lys Gly Gly Cys Gly
    130                 135                 140

Ser Tyr Gly Cys Ser Gln Cys Ser Cys Cys Lys Pro Cys Cys Cys Ser
145                 150                 155                 160

Ser Gly Cys Gly Ser Ser Cys Cys Gln Ser Ser Cys Cys Lys Pro Tyr
                165                 170                 175

Cys Cys Gln Ser Ser Cys Cys Lys Pro Cys Ser Cys Phe Ser Gly Cys
            180                 185                 190

Gly Ser Ser Cys Cys Gln Ser Ser Cys Tyr Lys Pro Cys Cys Cys Gln
        195                 200                 205

Ser Ser Cys Cys Val Pro Val Cys Cys Gln Cys Lys Ile
    210                 215                 220

<210>  33
<211>  129
<212>  PRT
<213>  Homo sapiens

<400>  33

Met Gly Cys Cys Gly Cys Ser Gly Gly Cys Gly Ser Gly Cys Gly Gly
1               5                   10                  15

Cys Gly Ser Gly Cys Gly Gly Cys Gly Ser Ser Cys Cys Val Pro Ile
            20                  25                  30

Cys Cys Cys Lys Pro Val Cys Cys Cys Val Pro Ala Cys Ser Cys Thr
        35                  40                  45

Ser Cys Gly Ser Cys Gly Gly Ser Lys Gly Cys Cys Gly Ser Cys Gly
    50                  55                  60

Gly Ser Lys Gly Gly Cys Gly Ser Cys Gly Gly Ser Lys Gly Gly Cys

65                  70                  75                  80

Gly Ser Cys Gly Cys Ser Gln Cys Ser Cys Cys Lys Pro Cys Tyr Cys
                85                  90                  95

Ser Ser Gly Cys Gly Ser Ser Cys Cys Gln Ser Ser Cys Cys Lys Pro
            100                 105                 110

Cys Cys Ser Gln Ala Ser Cys Cys Val Pro Ile Cys Cys Gln Cys Lys
        115                 120                 125

Ile

<210>  34
<211>  165
<212>  PRT
<213>  Homo sapiens

<400>  34

Met Gly Cys Cys Gly Cys Ser Glu Gly Cys Gly Ser Gly Cys Gly Gly
1               5                   10                  15

Cys Gly Ser Gly Cys Gly Gly Cys Gly Ser Gly Cys Gly Gly Cys Gly
            20                  25                  30

Ser Ser Cys Cys Val Pro Val Cys Cys Cys Lys Pro Val Cys Cys Cys
        35                  40                  45

Val Pro Ala Cys Ser Cys Ser Ser Cys Gly Ser Cys Gly Gly Ser Lys
    50                  55                  60

Gly Gly Cys Gly Ser Cys Gly Gly Ser Lys Gly Gly Cys Gly Ser Cys
65                  70                  75                  80

Gly Gly Ser Lys Gly Gly Cys Gly Ser Cys Gly Cys Ser Gln Cys Ser
                85                  90                  95

Cys Tyr Lys Pro Cys Cys Cys Ser Ser Gly Cys Gly Ser Ser Cys Cys
            100                 105                 110

Gln Ser Ser Cys Cys Lys Pro Cys Cys Cys Gln Ser Ser Cys Cys Lys
        115                 120                 125

Pro Cys Cys Cys Ser Ser Gly Cys Gly Ser Ser Cys Cys Gln Ser Ser
    130                 135                 140

Cys Cys Asn Pro Cys Cys Ser Gln Ser Ser Cys Cys Val Pro Val Cys
145                 150                 155                 160

Cys Gln Cys Lys Ile
                165

<210>  35
<211>  187
<212>  PRT
<213>  Homo sapiens

<400>  35

Met Gly Cys Cys Gly Cys Ser Gly Gly Cys Gly Ser Gly Cys Gly Gly
1               5                   10                  15

Cys Gly Ser Gly Cys Gly Gly Cys Gly Ser Ser Cys Cys Val Pro Ile
            20                  25                  30

Cys Cys Cys Lys Pro Val Cys Cys Cys Val Pro Ala Cys Ser Cys Ser
        35                  40                  45

Ser Cys Gly Ser Cys Gly Gly Ser Lys Gly Gly Arg Gly Ser Cys Gly
    50                  55                  60

Gly Ser Lys Gly Asp Cys Gly Ser Cys Gly Gly Ser Lys Gly Gly Cys
65                  70                  75                  80

Gly Ser Cys Gly Cys Ser Gln Cys Ser Cys Tyr Lys Pro Cys Cys Cys
                85                  90                  95

Ser Ser Gly Cys Gly Ser Ser Cys Cys Gln Ser Ser Cys Cys Lys Pro
            100                 105                 110

Cys Cys Ser Gln Ser Ser Cys Cys Lys Pro Cys Ser Cys Ser Ser Gly
        115                 120                 125

Cys Gly Ser Ser Cys Cys Gln Ser Ser Cys Cys Lys Pro Cys Cys Ser
    130                 135                 140

Gln Ser Ser Cys Cys Lys Pro Cys Cys Cys Ser Ser Gly Cys Gly Ser
145                 150                 155                 160

Ser Cys Cys Gln Ser Ser Cys Cys Lys Pro Cys Cys Ser Gln Ser Ser
                165                 170                 175

Cys Cys Val Pro Ile Cys Cys Gln Cys Lys Ile
            180                 185

<210>  36
<211>  169
<212>  PRT
<213>  Homo sapiens

<400>  36

Met Gly Cys Cys Gly Cys Ser Gly Gly Cys Gly Ser Ser Cys Gly Gly
1               5                   10                  15

Cys Asp Ser Ser Cys Gly Ser Cys Gly Ser Gly Cys Arg Gly Cys Gly
            20                  25                  30

Pro Ser Cys Cys Ala Pro Val Tyr Cys Cys Lys Pro Val Cys Cys Cys
        35                  40                  45

Val Pro Ala Cys Ser Cys Ser Ser Cys Gly Lys Arg Gly Cys Gly Ser
    50                  55                  60

Cys Gly Gly Ser Lys Gly Gly Cys Gly Ser Cys Gly Cys Ser Gln Cys
65                  70                  75                  80

Ser Cys Cys Lys Pro Cys Cys Cys Ser Ser Gly Cys Gly Ser Ser Cys
                85                  90                  95

Cys Gln Cys Ser Cys Cys Lys Pro Tyr Cys Ser Gln Cys Ser Cys Cys
            100                 105                 110

Lys Pro Cys Cys Ser Ser Ser Gly Arg Gly Ser Ser Cys Cys Gln Ser
        115                 120                 125

Ser Cys Cys Lys Pro Cys Cys Ser Ser Ser Gly Cys Gly Ser Ser Cys
    130                 135                 140

Cys Gln Ser Ser Cys Cys Lys Pro Cys Cys Ser Gln Ser Arg Cys Cys
145                 150                 155                 160

Val Pro Val Cys Tyr Gln Cys Lys Ile
                165

<210>  37
<211>  202
<212>  PRT
<213>  Homo sapiens

<400>  37

Met Gly Cys Cys Gly Cys Ser Gly Gly Cys Gly Ser Gly Cys Gly Gly
1               5                   10                  15

Cys Gly Ser Gly Cys Gly Gly Cys Gly Ser Gly Cys Gly Gly Tyr Gly
            20                  25                  30

Ser Gly Cys Gly Gly Cys Gly Ser Ser Cys Cys Val Pro Val Cys Cys
        35                  40                  45

Cys Lys Pro Val Cys Cys Cys Val Pro Ala Cys Ser Cys Ser Ser Cys
    50                  55                  60

Gly Ser Cys Gly Gly Ser Lys Gly Asp Cys Gly Ser Cys Gly Gly Ser
65                  70                  75                  80

Lys Gly Gly Cys Gly Ser Cys Gly Gly Ser Lys Gly Gly Cys Gly Ser

85                  90                  95

Cys Gly Gly Ser Lys Gly Gly Cys Gly Ser Cys Gly Gly Ser Lys Gly
            100                 105                 110

Gly Cys Gly Ser Cys Gly Gly Ser Lys Gly Gly Cys Gly Ser Cys Gly
        115                 120                 125

Gly Ser Lys Gly Gly Cys Gly Ser Cys Gly Cys Ser Gln Cys Asn Cys
    130                 135                 140

Cys Lys Pro Cys Cys Cys Ser Ser Gly Cys Gly Ser Cys Cys Gln Ser
145                 150                 155                 160

Ser Cys Cys Asn Pro Cys Cys Cys Gln Ser Ser Cys Cys Val Pro Val
                165                 170                 175

Cys Cys Gln Ser Ser Cys Cys Lys Pro Cys Cys Cys Gln Ser Ser Cys
            180                 185                 190

Cys Val Pro Val Cys Cys Gln Cys Lys Ile
        195                 200

<210>  38
<211>  156
<212>  PRT
<213>  Homo sapiens

<400>  38

Met Gly Cys Cys Gly Cys Ser Gly Gly Cys Gly Ser Gly Cys Gly Gly
1               5                   10                  15

Cys Gly Ser Gly Ser Gly Gly Cys Gly Ser Gly Cys Gly Gly Cys Gly
            20                  25                  30

Ser Ser Cys Cys Val Pro Ile Cys Cys Cys Lys Pro Val Cys Cys Cys
        35                  40                  45

Val Pro Ala Cys Ser Cys Ser Ser Cys Gly Ser Cys Gly Gly Ser Lys
    50                  55                  60

Gly Gly Cys Gly Ser Cys Gly Ser Ser Lys Gly Gly Cys Gly Ser Cys
65                  70                  75                  80

Gly Cys Ser Gln Ser Asn Cys Cys Lys Pro Cys Cys Ser Ser Ser Gly
                85                  90                  95

Cys Gly Ser Phe Cys Cys Gln Ser Ser Cys Ser Lys Pro Cys Cys Cys
            100                 105                 110

Gln Ser Ser Cys Cys Gln Ser Ser Cys Cys Lys Pro Cys Cys Cys Gln
        115                 120                 125

Ser Ser Cys Cys Gln Ser Ser Cys Phe Lys Pro Cys Cys Cys Gln Ser
    130                 135                 140

Ser Cys Cys Val Pro Val Cys Cys Gln Cys Lys Ile
145                 150                 155

<210>  39
<211>  87
<212>  PRT
<213>  Homo sapiens

<400>  39

Met Thr Arg Tyr Phe Cys Cys Gly Ser Tyr Phe Pro Gly Tyr Pro Ile
1               5                   10                  15

Tyr Gly Thr Asn Phe His Gly Thr Phe Arg Ala Thr Pro Leu Asn Cys
            20                  25                  30

Val Val Pro Leu Gly Ser Pro Leu Asn Tyr Gly Cys Gly Cys Asn Gly
        35                  40                  45

Tyr Ser Ser Leu Gly Tyr Ser Phe Gly Gly Ser Asn Ile Asn Asn Leu
    50                  55                  60

Gly Gly Cys Tyr Gly Gly Ser Phe Tyr Arg Pro Trp Gly Ser Gly Ser
65                  70                  75                  80

Gly Phe Gly Tyr Ser Thr Tyr
                85

<210>  40
<211>  63
<212>  PRT
<213>  Homo sapiens

<400>  40

Met Leu Cys Asp Asn Phe Pro Gly Ala Val Phe Pro Gly Cys Tyr Trp
1               5                   10                  15

Gly Ser Tyr Gly Tyr Pro Leu Gly Tyr Ser Val Gly Cys Gly Tyr Gly
            20                  25                  30

Ser Thr Tyr Ser Pro Val Gly Tyr Gly Phe Gly Tyr Gly Tyr Asn Gly
        35                  40                  45

Cys Gly Ala Phe Gly Tyr Arg Arg Tyr Ser Pro Phe Ala Leu Tyr
    50                  55                  60

<210>  41
<211>  174
<212>  PRT
<213>  Homo sapiens

<400> 41

<210> 42
<211> 159
<212> PRT
<213> Homo sapiens

<400> 42

Met Thr His Cys Cys Ser Pro Cys Cys Gln Pro Thr Cys Cys Arg Thr
1               5                   10                  15

Thr Cys Trp Gln Pro Thr Thr Val Thr Thr Cys Ser Ser Thr Pro Cys
            20                  25                  30

Cys Gln Pro Ser Cys Cys Val Ser Ser Cys Cys Gln Pro Cys Cys His
        35                  40                  45

Pro Thr Cys Cys Gln Asn Thr Cys Cys Arg Thr Thr Cys Cys Gln Pro
    50                  55                  60

Ile Cys Val Thr Ser Cys Cys Gln Pro Ser Cys Cys Ser Thr Pro Cys
65                  70                  75                  80

Cys Gln Pro Thr Cys Cys Gly Ser Ser Cys Gly Gln Ser Ser Ser Cys
                85                  90                  95

Ala Pro Val Tyr Cys Arg Arg Thr Cys Tyr His Pro Thr Ser Val Cys
            100                 105                 110

Leu Pro Gly Cys Leu Asn Gln Ser Cys Gly Ser Asn Cys Cys Gln Pro
        115                 120                 125

Cys Cys Arg Pro Ala Cys Cys Glu Thr Thr Cys Cys Arg Thr Thr Cys
    130                 135                 140

Phe Gln Pro Thr Cys Val Tyr Ser Cys Cys Gln Pro Ser Cys Cys
145                 150                 155

<210>  43
<211>  154
<212>  PRT
<213>  Homo sapiens

<400>  43

Met Thr His Cys Cys Ser Pro Cys Cys Gln Pro Thr Cys Cys Arg Thr
1               5                   10                  15

Thr Cys Cys Arg Thr Thr Cys Trp Lys Pro Thr Thr Val Thr Thr Cys
            20                  25                  30

Ser Ser Thr Pro Cys Cys Gln Pro Ser Cys Cys Val Ser Ser Cys Cys
        35                  40                  45

Gln Pro Cys Cys Arg Pro Thr Cys Cys Gln Asn Thr Cys Cys Gln Pro
    50                  55                  60

Thr Cys Val Thr Ser Cys Cys Gln Pro Ser Cys Cys Ser Thr Pro Cys
65                  70                  75                  80

Cys Gln Pro Thr Cys Cys Gly Ser Ser Cys Asp Gln Ser Ser Ser Cys
                85                  90                  95

Ala Pro Val Tyr Cys Arg Arg Thr Cys Tyr Tyr Pro Thr Thr Val Cys
            100                 105                 110

Leu Pro Gly Cys Leu Asn Gln Ser Cys Gly Ser Asn Cys Cys Gln Pro
        115                 120                 125

Cys Cys Arg Pro Ala Cys Cys Glu Thr Thr Cys Phe Gln Pro Thr Cys
    130                 135                 140

Val Tyr Ser Cys Cys Gln Pro Phe Cys Cys
145                 150

<210>  44
<211>  111
<212>  PRT
<213>  Homo sapiens

<400>  44

Thr Thr Cys Cys Gln Pro Thr Cys Leu Thr Ser Cys Cys Gln Pro Ser
1               5                   10                  15

Cys Cys Ser Thr Thr Cys Cys Gln Pro Ile Cys Cys Gly Ser Ser Cys
            20                  25                  30

Cys Gly Gln Thr Ser Cys Gly Ser Ser Cys Gly Gln Ser Ser Ser Cys
        35                  40                  45

Ala Pro Val Tyr Cys Arg Arg Thr Cys Tyr Tyr Pro Thr Thr Val Cys
    50                  55                  60

Leu Pro Gly Cys Leu Asn Gln Ser Cys Gly Ser Ser Cys Cys Gln Pro
65                  70                  75                  80

Cys Cys Arg Pro Ala Cys Cys Glu Thr Thr Cys Cys Arg Thr Thr Cys
                85                  90                  95

Phe Gln Pro Thr Cys Val Ser Ser Cys Cys Gln Pro Ser Cys Cys
            100                 105                 110

<210>  45
<211>  159
<212>  PRT
<213>  Homo sapiens

<400>  45

Met Thr His Cys Cys Ser Pro Cys Cys Gln Pro Thr Cys Cys Arg Thr
1               5                   10                  15

Thr Cys Trp Lys Pro Thr Thr Val Thr Thr Cys Ser Ser Thr Pro Cys
            20                  25                  30

Cys Gln Pro Ser Cys Cys Val Ser Ser Cys Cys Gln Pro Cys Cys Arg
        35                  40                  45

Pro Thr Cys Cys Gln Asn Thr Cys Cys Gln Pro Ile Cys Val Thr Ser
    50                  55                  60

Cys Cys Gln Pro Ser Cys Cys Ser Thr Pro Cys Cys Gln Pro Thr Cys
65                  70                  75                  80

Cys Gly Gln Thr Ser Cys Gly Ser Ser Cys Gly Gln Ser Ser Ser Cys

                85                  90                  95

Ala Pro Val Tyr Cys Arg Arg Thr Cys Tyr His Pro Thr Thr Val Cys
            100                 105                 110

Leu Pro Gly Cys Leu Asn Gln Ser Cys Gly Ser Ser Cys Cys Gln Pro
        115                 120                 125

Cys Cys Arg Pro Ala Cys Cys Glu Thr Thr Cys Cys Arg Thr Thr Cys
    130                 135                 140

Phe Gln Pro Thr Cys Val Tyr Ser Cys Cys Gln Pro Ser Cys Cys
145                 150                 155

<210> 46
<211> 154
<212> PRT
<213> Homo sapiens

<400> 46

Met Thr His Cys Cys Ser Pro Cys Cys Gln Pro Thr Cys Cys Arg Thr
1               5                   10                  15

Thr Cys Cys Arg Thr Thr Cys Trp Lys Pro Thr Thr Val Thr Thr Cys
            20                  25                  30

Ser Ser Thr Pro Cys Cys Gln Pro Ser Cys Cys Val Ser Ser Cys Cys
        35                  40                  45

Gln Pro Cys Cys Arg Pro Ala Cys Cys Gln Asn Thr Cys Cys Arg Thr
    50                  55                  60

Thr Cys Cys Gln Pro Thr Cys Leu Ser Ser Cys Cys Gly Gln Thr Ser
65                  70                  75                  80

Cys Gly Ser Ser Cys Gly Gln Ser Ser Ser Cys Ala Pro Val Tyr Cys
                85                  90                  95

Arg Arg Thr Cys Tyr Tyr Pro Thr Thr Val Cys Leu Pro Gly Cys Leu
            100                 105                 110

Asn Gln Ser Cys Gly Ser Ser Cys Cys Gln Pro Cys Cys Arg Pro Ala
        115                 120                 125

Cys Cys Glu Thr Thr Cys Cys Arg Thr Thr Cys Phe Gln Pro Thr Cys
    130                 135                 140

Val Ser Ser Cys Cys Gln Pro Ser Cys Cys
145                 150

<210> 47
<211> 282

<212>  PRT
<213>  Homo sapiens

<400>  47

Met Ala Ala Ser Thr Met Ser Val Cys Ser Ser Ala Cys Ser Asp Ser
1               5                   10                  15

Trp Gln Val Asp Ala Cys Pro Glu Ser Cys Cys Glu Pro His Cys Cys
            20                  25                  30

Ala Leu Ser Cys Cys Ala Pro Ala Pro Cys Leu Thr Leu Val Cys Thr
        35                  40                  45

Pro Val Ser Arg Val Ser Ser Pro Cys Cys Gln Ala Ala Cys Glu Pro
    50                  55                  60

Ser Pro Cys Gln Ser Gly Cys Thr Ser Ser Cys Thr Pro Ser Cys Cys
65                  70                  75                  80

Gln Gln Ser Ser Cys Gln Pro Ala Cys Cys Thr Ser Ser Pro Cys Gln
                85                  90                  95

Gln Ala Cys Cys Val Pro Val Cys Cys Lys Pro Val Cys Cys Leu Pro
            100                 105                 110

Thr Cys Ser Lys Asp Ser Ser Ser Cys Cys Gln Gln Ser Ser Cys Gln
        115                 120                 125

Pro Thr Cys Cys Ala Ser Ser Ser Ser Gln Gln Ser Cys Cys Val Pro
    130                 135                 140

Val Cys Cys Lys Pro Val Cys Tyr Val Pro Thr Cys Ser Glu Asp Ser
145                 150                 155                 160

Ser Ser Cys Cys Gln Gln Ser Ser Cys His Pro Ala Cys Cys Thr Ser
                165                 170                 175

Ser Pro Cys Gln Gln Ala Cys Cys Val Pro Val Arg Cys Lys Pro Val
            180                 185                 190

Cys Cys Lys Pro Ile Cys Cys Val Pro Val Cys Ser Gly Ala Ser Thr
        195                 200                 205

Ser Cys Cys Gln Gln Ser Ser Cys Gln Pro Ala Cys Cys Thr Thr Ser
    210                 215                 220

Cys Cys Arg Pro Ser Ser Ser Val Ser Leu Leu Cys Arg Pro Val Cys
225                 230                 235                 240

Arg Pro Ala Cys Cys Met Pro Val Ser Ser Cys Cys Ala Pro Ala Ser
                245                 250                 255

Ser Cys Gln Ala Ser Cys Cys Arg Pro Ala Ser Cys Val Ser Leu Leu
            260                 265                 270

Cys Arg Pro Ala Cys Ser Arg Pro Ala Cys
        275                 280

<210>  48
<211>  255
<212>  PRT
<213>  Homo sapiens

<400>  48

Met Ala Ala Ser Thr Met Ser Ile Cys Ser Ser Ala Cys Thr Asn Ser
1               5                   10                  15

Trp Gln Val Asp Asp Cys Pro Glu Ser Cys Cys Glu Leu Pro Cys Gly
            20                  25                  30

Thr Pro Ser Cys Cys Ala Pro Ala Pro Cys Leu Thr Leu Val Cys Thr
        35                  40                  45

Pro Val Ser Cys Val Ser Ser Pro Cys Cys Gln Ala Ala Cys Glu Pro
    50                  55                  60

Ser Ala Cys Gln Ser Gly Cys Thr Ser Ser Cys Thr Pro Ser Cys Cys
65                  70                  75                  80

Gln Gln Ser Ser Cys Gln Pro Ala Cys Cys Thr Ser Ser Pro Cys Gln
                85                  90                  95

Gln Ala Cys Cys Val Pro Val Cys Cys Lys Pro Val Cys Cys Val Pro
            100                 105                 110

Val Cys Cys Gly Ala Ser Ser Cys Cys Gln Gln Ser Ser Cys Gln Pro
        115                 120                 125

Ala Cys Cys Ala Ser Ser Ser Cys Gln Gln Ser Cys Arg Val Pro Val
    130                 135                 140

Cys Cys Lys Ala Val Cys Cys Val Pro Thr Cys Ser Glu Ser Ser Ser
145                 150                 155                 160

Ser Cys Cys Gln Gln Ser Ser Cys Gln Pro Ala Cys Cys Thr Ser Ser
                165                 170                 175

Pro Cys Gln Gln Ser Cys Cys Val Ser Val Cys Cys Lys Pro Val Cys
            180                 185                 190

Cys Lys Ser Ile Cys Cys Val Pro Val Cys Ser Gly Ala Ser Ser Pro
        195                 200                 205

Cys Cys Gln Gln Ser Ser Cys Gln Pro Ala Cys Cys Thr Ser Ser Cys
    210                 215                 220

Cys Arg Pro Ser Ser Ser Val Ser Leu Leu Cys Arg Pro Val Cys Ser
225                 230                 235                 240

Arg Pro Ala Ser Cys Ser Phe Ser Ser Gly Gln Lys Ser Ser Cys
                245                 250                 255

<210>  49
<211>  221
<212>  PRT
<213>  Homo sapiens

<400>  49

Met Ala Thr Ser Thr Met Ser Val Cys Ser Ser Ala Tyr Ser Asp Ser
1               5                   10                  15

Trp Gln Val Asp Ala Cys Pro Glu Ser Cys Cys Glu Pro Pro Cys Cys
            20                  25                  30

Ala Thr Ser Cys Cys Ala Pro Ala Pro Cys Leu Thr Leu Val Cys Thr
        35                  40                  45

Pro Val Ser Cys Val Ser Ser Pro Cys Cys Gln Ala Ala Cys Glu Pro
    50                  55                  60

Ser Pro Cys Gln Ser Gly Cys Thr Ser Ser Cys Thr Pro Ser Cys Cys
65                  70                  75                  80

Gln Gln Ser Ser Cys Gln Pro Ala Cys Cys Thr Ser Ser Pro Cys Gln
                85                  90                  95

Gln Ala Cys Cys Val Pro Val Cys Cys Lys Pro Val Cys Cys Val Pro
            100                 105                 110

Val Cys Cys Lys Pro Val Cys Cys Lys Pro Ile Cys Cys Val Pro Val
        115                 120                 125

Cys Ser Gly Ala Ser Ser Ser Cys Cys Gln Gln Ser Ser Arg Gln Pro
    130                 135                 140

Ala Cys Cys Thr Thr Ser Cys Cys Arg Pro Ser Ser Ser Val Ser Leu
145                 150                 155                 160

Leu Cys Arg Pro Val Cys Arg Ser Thr Cys Cys Val Pro Ile Pro Ser
                165                 170                 175

Cys Cys Ala Pro Ala Ser Thr Cys Gln Pro Ser Cys Cys Arg Pro Ala
            180                 185                 190

Ser Cys Val Ser Leu Leu Cys Arg Pro Thr Cys Ser Arg Leu Ser Ser

195                 200                 205

Ala Cys Cys Gly Leu Ser Ser Gly Gln Lys Ser Ser Cys
    210                 215                 220

<210>  50
<211>  401
<212>  PRT
<213>  Homo sapiens

<400>  50

Met Ser Val Cys Ser Ser Asp Leu Ser Tyr Ser Ser Arg Val Cys Leu
1               5                   10                  15

Pro Gly Ser Cys Asp Ser Cys Ser Asp Ser Trp Gln Val Asp Asp Cys
            20                  25                  30

Pro Glu Ser Cys Cys Glu Pro Pro Cys Cys Ala Pro Ser Cys Cys Ala
        35                  40                  45

Pro Ala Pro Cys Leu Ser Leu Val Cys Thr Pro Val Ser Arg Val Ser
    50                  55                  60

Ser Pro Cys Cys Pro Val Thr Cys Glu Pro Ser Pro Cys Gln Ser Gly
65                  70                  75                  80

Cys Thr Ser Ser Cys Thr Pro Ser Cys Cys Gln Gln Ser Ser Cys Gln
                85                  90                  95

Leu Ala Cys Cys Ala Ser Ser Pro Cys Gln Gln Ala Cys Cys Val Pro
            100                 105                 110

Val Cys Cys Lys Thr Val Cys Cys Lys Pro Val Cys Cys Val Pro Val
        115                 120                 125

Cys Cys Gly Asp Ser Ser Cys Cys Gln Gln Ser Ser Cys Gln Ser Ala
    130                 135                 140

Cys Cys Thr Ser Ser Pro Cys Gln Gln Ala Cys Cys Val Pro Ile Cys
145                 150                 155                 160

Cys Lys Pro Val Cys Ser Gly Ile Ser Ser Ser Cys Cys Gln Gln Ser
                165                 170                 175

Ser Cys Val Ser Cys Val Ser Ser Pro Cys Cys Gln Ala Val Cys Glu
            180                 185                 190

Pro Ser Pro Cys Gln Ser Gly Cys Ile Ser Ser Cys Thr Pro Ser Cys
        195                 200                 205

Cys Gln Gln Ser Ser Cys Gln Pro Ala Cys Cys Thr Ser Ser Ser Cys
    210                 215                 220

Gln Gln Ala Cys Cys Val Pro Val Cys Cys Lys Thr Val Cys Cys Lys
225                 230                 235                 240

Pro Val Cys Ser Glu Asp Ser Ser Ser Cys Cys Gln Gln Ser Ser Cys
                245                 250                 255

Gln Pro Ala Cys Cys Thr Ser Ser Pro Cys Gln Gln Ala Cys Cys Val
            260                 265                 270

Pro Val Cys Cys Lys Pro Val Cys Cys Lys Pro Val Gly Ser Val Pro
        275                 280                 285

Ile Cys Ser Gly Ala Ser Ser Leu Cys Cys Gln Gln Ser Ser Cys Gln
    290                 295                 300

Pro Ala Cys Cys Thr Ser Ser Gln Ser Gln Gln Gly Cys Cys Val Pro
305                 310                 315                 320

Val Cys Cys Lys Pro Val Ser Cys Val Pro Val Cys Ser Gly Ala Ser
                325                 330                 335

Ser Ser Cys Cys Gln Gln Ser Ser Cys Gln Pro Ala Cys Cys Thr Thr
            340                 345                 350

Ser Cys Cys Arg Pro Ser Ser Ser Val Ser Leu Leu Cys Arg Pro Val
        355                 360                 365

Cys Arg Pro Ala Cys Cys Val Pro Val Pro Ser Cys Cys Ala Pro Thr
    370                 375                 380

Ser Ser Cys Gln Pro Ser Cys Cys Arg Pro Ala Ser Cys Val Ser Leu
385                 390                 395                 400

Leu

<210>  51
<211>  271
<212>  PRT
<213>  Homo sapiens

<400>  51

Met Ala Ala Cys Thr Met Ser Val Cys Ser Ser Ala Cys Ser Asp Ser
1               5                   10                  15

Trp Arg Val Asp Asp Cys Pro Glu Ser Cys Cys Glu Pro Pro Cys Gly
            20                  25                  30

Thr Ala Pro Cys Leu Thr Leu Val Cys Thr Pro Val Ser Cys Val Ser
        35                  40                  45

Ser Pro Cys Cys Gln Ala Ala Cys Glu Pro Ser Pro Cys Gln Ser Gly
    50                  55                  60

Cys Thr Ser Ser Cys Thr Pro Ser Cys Cys Gln Pro Ala Cys Cys Ala
65                  70                  75                  80

Ser Ser Pro Cys Gln Gln Ala Cys Cys Val Pro Val Cys Cys Lys Pro
                85                  90                  95

Val Cys Cys Leu Pro Thr Cys Ser Lys Asp Ser Ser Ser Cys Cys Gln
            100                 105                 110

Gln Ser Ser Cys Gln Pro Thr Cys Cys Ala Ser Ser Ser Cys Gln Gln
        115                 120                 125

Ser Cys Cys Val Pro Val Cys Cys Lys Pro Val Cys Cys Val Pro Thr
    130                 135                 140

Cys Ser Glu Asp Ser Ser Ser Cys Cys Gln His Ser Ser Cys Gln Pro
145                 150                 155                 160

Thr Cys Cys Thr Ser Ser Pro Cys Gln Gln Ser Cys Tyr Val Pro Val
                165                 170                 175

Cys Cys Lys Pro Val Cys Phe Lys Pro Ile Cys Cys Val Pro Val Cys
            180                 185                 190

Ser Gly Ala Ser Thr Ser Cys Cys Gln Gln Ser Ser Cys Gln Pro Ala
        195                 200                 205

Cys Cys Thr Thr Ser Cys Cys Arg Pro Ser Ser Ser Val Ser Leu Leu
    210                 215                 220

Cys Arg Pro Ile Cys Arg Pro Ala Cys Cys Val Pro Ile Ser Ser Cys
225                 230                 235                 240

Cys Ala Pro Ala Ser Ser Tyr Gln Ala Ser Cys Cys Arg Pro Ala Ser
                245                 250                 255

Cys Val Ser Leu Leu Cys Arg Pro Ala Cys Ser Arg Leu Ala Cys
            260                 265                 270

<210> 52
<211> 365
<212> PRT
<213> Homo sapiens

<400> 52

Met Ala Ala Ser Thr Met Ser Val Cys Ser Ser Asp Leu Ser Tyr Gly
1               5                   10                  15

Ser Arg Val Cys Leu Pro Gly Ser Cys Asp Ser Cys Ser Asp Ser Trp
            20                  25                  30

Gln Val Asp Asp Cys Pro Glu Ser Cys Cys Glu Pro Pro Cys Cys Ala
        35                  40                  45

Pro Ala Pro Cys Leu Ser Leu Val Cys Thr Pro Val Ser Arg Val Ser
    50                  55                  60

Ser Pro Cys Cys Pro Val Thr Cys Glu Pro Ser Pro Cys Gln Ser Gly
65                  70                  75                  80

Cys Thr Ser Ser Cys Thr Pro Ser Cys Cys Gln Gln Ser Ser Cys Gln
                85                  90                  95

Leu Ala Cys Cys Ala Ser Ser Pro Cys Gln Gln Ala Cys Cys Val Pro
            100                 105                 110

Val Cys Cys Lys Thr Val Cys Cys Lys Pro Val Cys Cys Val Ser Val
        115                 120                 125

Cys Cys Gly Asp Ser Ser Cys Cys Gln Gln Ser Ser Cys Gln Ser Ala
    130                 135                 140

Cys Cys Thr Ser Ser Pro Cys Gln Gln Ala Cys Cys Val Pro Val Cys
145                 150                 155                 160

Cys Lys Pro Val Cys Ser Gly Ile Ser Ser Ser Cys Cys Gln Gln Ser
                165                 170                 175

Ser Cys Val Ser Cys Val Ser Ser Pro Cys Cys Gln Ala Val Cys Glu
            180                 185                 190

Pro Ser Pro Cys Gln Ser Gly Cys Thr Ser Ser Cys Thr Pro Ser Cys
        195                 200                 205

Cys Gln Gln Ser Ser Cys Gln Pro Thr Cys Cys Thr Ser Ser Pro Cys
    210                 215                 220

Gln Gln Ala Cys Cys Val Pro Val Cys Cys Val Pro Val Cys Cys Val
225                 230                 235                 240

Pro Thr Cys Ser Glu Asp Ser Ser Ser Cys Cys Gln Gln Ser Ser Cys
                245                 250                 255

Gln Pro Ala Cys Cys Thr Ser Ser Pro Cys Gln His Ala Cys Cys Val
            260                 265                 270

Pro Val Cys Ser Gly Ala Ser Thr Ser Cys Cys Gln Gln Ser Ser Cys
        275                 280                 285

Gln Pro Ala Cys Cys Thr Ala Ser Cys Cys Arg Pro Ser Ser Ser Val
    290                 295                 300

Ser Leu Leu Cys His Pro Val Cys Lys Ser Thr Cys Cys Val Pro Val
305                 310                 315                 320

Pro Ser Cys Gly Ala Ser Ala Ser Ser Cys Gln Pro Ser Cys Cys Arg
                325                 330                 335

Thr Ala Ser Cys Val Ser Leu Leu Cys Arg Pro Met Cys Ser Arg Pro
            340                 345                 350

Ala Cys Tyr Ser Leu Cys Ser Gly Gln Lys Ser Ser Cys
        355                 360                 365

<210>  53
<211>  375
<212>  PRT
<213>  Homo sapiens

<400>  53

Met Ala Ala Ser Thr Met Ser Val Cys Ser Ser Asp Leu Ser Tyr Gly
1               5                   10                  15

Ser Arg Val Cys Leu Pro Gly Ser Cys Asp Ser Cys Ser Asp Ser Trp
            20                  25                  30

Gln Val Asp Asp Cys Pro Glu Ser Cys Cys Glu Pro Pro Cys Cys Ala
        35                  40                  45

Pro Ser Cys Cys Ala Pro Ala Pro Cys Leu Ser Leu Val Cys Thr Pro
    50                  55                  60

Val Ser Arg Val Ser Ser Pro Cys Cys Pro Val Thr Cys Glu Pro Ser
65                  70                  75                  80

Pro Cys Gln Ser Gly Cys Thr Ser Ser Cys Thr Pro Ser Cys Cys Gln
                85                  90                  95

Gln Ser Ser Cys Gln Leu Ala Cys Cys Ala Ser Ser Pro Cys Gln Gln
            100                 105                 110

Ala Cys Cys Met Pro Val Cys Cys Lys Thr Val Cys Cys Lys Pro Val
        115                 120                 125

Tyr Cys Val Pro Val Cys Ser Gly Asp Ser Ser Cys Cys Gln Gln Ser
    130                 135                 140

Ser Cys Gln Ser Ala Cys Cys Thr Ser Ser Pro Cys Gln Gln Ala Cys
145                 150                 155                 160

Cys Val Pro Ile Cys Cys Lys Pro Val Cys Ser Gly Ile Ser Ser Ser

165                 170                 175

Cys Cys Gln Gln Ser Ser Cys Val Ser Cys Val Ser Ser Pro Cys Cys
            180                 185                 190

Gln Ala Val Cys Glu Pro Ser Pro Cys Gln Ser Gly Cys Ile Ser Ser
        195                 200                 205

Cys Thr Pro Ser Cys Cys Gln Gln Ser Ser Cys Gln Pro Ala Cys Cys
    210                 215                 220

Thr Ser Ser Pro Cys Gln Gln Ala Cys Cys Val Pro Val Cys Cys Lys
225                 230                 235                 240

Pro Val Cys Cys Val Pro Thr Cys Ser Asp Asp Ser Gly Ser Cys Cys
                245                 250                 255

Gln Pro Ala Cys Cys Thr Ser Ser Gln Ser Gln Gln Gly Cys Cys Val
            260                 265                 270

Pro Val Cys Cys Lys Pro Val Cys Cys Val Pro Val Cys Ser Gly Ala
        275                 280                 285

Ser Ser Ser Cys Cys Gln Gln Ser Ser Cys Gln Pro Ala Cys Cys Thr
    290                 295                 300

Thr Ser Cys Cys Arg Pro Ser Ser Ser Val Ser Leu Leu Cys Arg Pro
305                 310                 315                 320

Val Cys Arg Pro Thr Cys Cys Val Pro Val Pro Ser Cys Cys Ala Pro
                325                 330                 335

Thr Ser Ser Cys Gln Ala Ser Cys Cys Arg Pro Ala Ser Cys Val Ser
            340                 345                 350

Leu Leu Cys Arg Pro Ala Cys Ser Arg Pro Ala Cys Cys Gly Pro Thr
        355                 360                 365

Ser Thr Gln Lys Ser Ser Cys
    370                 375

<210>  54
<211>  259
<212>  PRT
<213>  Homo sapiens

<400>  54

Met Ala Asp Ala Cys Cys Thr Arg Thr Tyr Val Ile Ala Ala Ser Thr
1               5                   10                  15

Met Ser Val Cys Ser Ser Asp Val Gly His Val Ser Arg Val Ser Ser
            20                  25                  30

Pro Ser Thr Cys Thr Gly Ser Ser Trp Gln Val Asp Asn Cys Gln Glu
        35                  40                  45

Ser Cys Cys Glu Pro Arg Ser Cys Ala Ser Ser Cys Cys Thr Pro Ser
    50                  55                  60

Cys Cys Ala Pro Ala Pro Cys Leu Ala Leu Val Cys Ala Pro Val Ser
65                  70                  75                  80

Cys Glu Pro Ser Pro Cys Gln Ser Gly Cys Thr Asp Ser Cys Thr Pro
                85                  90                  95

Ser Cys Cys Gln Gln Ser Ser Cys Gln Pro Ala Cys Cys Thr Ser Ser
            100                 105                 110

Pro Cys Gln Gln Ala Cys Cys Val Pro Val Cys Cys Lys Ser Asn Cys
        115                 120                 125

Cys Lys Pro Val Cys Cys Val Ser Ile Cys Ser Gly Ala Ser Ser Pro
    130                 135                 140

Cys Cys Gln Gln Ser Ser Cys Gln Ser Ala Cys Cys Thr Phe Ser Pro
145                 150                 155                 160

Cys Gln Gln Ala Cys Cys Val Pro Ile Cys Cys Lys Pro Ile Cys Cys
                165                 170                 175

Val Pro Val Cys Ser Gly Ala Ser Ser Leu Cys Cys Gln Lys Ser Ser
            180                 185                 190

Cys Gln Pro Ala Cys Cys Thr Thr Ser Cys Cys Arg Pro Ser Ser Ser
        195                 200                 205

Val Ser Leu Leu Cys Arg Pro Val Cys Arg Pro Ala Cys Cys Val Pro
    210                 215                 220

Val Pro Ser Cys Cys Val Pro Ala Ser Ser Cys Gln Pro Ser Cys Cys
225                 230                 235                 240

His Pro Ala Ser Cys Leu Ser Phe Leu Cys Arg Pro Ala Cys Ser Arg
                245                 250                 255

Leu Ala Cys

<210> 55
<211> 292
<212> PRT
<213> Homo sapiens

<400> 55

Met Ala Ala Ser Thr Met Ser Ile Arg Ser Ser Ala Tyr Ser Asp Ser
1               5                   10                  15

Trp Gln Val Asp Asp Cys Pro Glu Ser Cys Cys Glu Pro Pro Cys Cys
            20                  25                  30

Ala Thr Ser Cys Cys Ala Pro Ala Pro Cys Leu Thr Leu Val Cys Thr
        35                  40                  45

Pro Val Ser Arg Val Ser Ser Pro Cys Cys Gln Val Thr Cys Glu Pro
    50                  55                  60

Ser Pro Cys Gln Ser Gly Cys Thr Ser Ser Cys Thr Pro Ser Cys Cys
65                  70                  75                  80

Gln Gln Ser Ser Cys Gln Pro Ala Tyr Cys Thr Ser Ser Pro Cys Gln
                85                  90                  95

Gln Ala Cys Cys Val Pro Val Cys Cys Lys Pro Val Cys Cys Val Pro
            100                 105                 110

Val Cys Cys Gly Ala Ser Ser Cys Cys Gln Gln Ser Ser Tyr Gln Pro
        115                 120                 125

Ala Cys Cys Ala Ser Ser Ser Cys Gln Pro Ala Cys Cys Val Pro Val
    130                 135                 140

Cys Cys Lys Pro Val Cys Cys Ala Pro Thr Cys Ser Glu Asp Ser Tyr
145                 150                 155                 160

Ser Cys Cys Gln Gln Ser Ser Cys Gln Pro Ala Cys Cys Thr Ser Ser
                165                 170                 175

Pro Cys Gln Gln Ser Tyr Cys Val Pro Val Cys Cys Lys Pro Val Cys
            180                 185                 190

Cys Lys Pro Ile Cys Cys Val Pro Val Cys Ser Gly Ala Ser Ser Leu
        195                 200                 205

Cys Cys Gln Gln Ser Gly Cys Gln Pro Ala Cys Cys Thr Thr Ser Cys
    210                 215                 220

Cys Arg Pro Ser Ser Ser Val Ser Leu Leu Cys Arg Pro Val Cys Arg
225                 230                 235                 240

Pro Ala Cys Cys Val Pro Val Ser Ser Cys Cys Ala Pro Thr Ser Ser
                245                 250                 255

Arg Gln Pro Ser Tyr Cys Arg Gln Ala Ser Cys Val Ser Leu Leu Cys
            260                 265                 270

Arg Pro Val Cys Ser Arg Pro Ala Cys Tyr Ser Phe Ser Ser Gly Gln
        275                 280                 285

Lys Ser Ser Cys
    290

<210>  56
<211>  251
<212>  PRT
<213>  Homo sapiens

<400>  56

Met Ala Ala Ser Thr Met Ser Ile Cys Ser Ser Ala Cys Thr Asp Ser
1               5                   10                  15

Trp Arg Val Val Asp Cys Pro Glu Ser Cys Cys Glu Pro Cys Cys Cys
            20                  25                  30

Ala Pro Ala Pro Ser Leu Thr Leu Val Cys Thr Pro Val Ser Cys Val
        35                  40                  45

Ser Ser Pro Cys Cys Gln Thr Ala Cys Glu Pro Ser Ala Cys Gln Ser
    50                  55                  60

Gly Tyr Thr Ser Ser Cys Thr Thr Pro Cys Tyr Gln Gln Ser Ser Cys
65                  70                  75                  80

Gln Pro Asp Cys Cys Thr Ser Ser Pro Cys Gln Gln Ala Cys Cys Val
                85                  90                  95

Pro Val Cys Cys Val Pro Val Cys Cys Val Pro Val Cys Asn Lys Pro
            100                 105                 110

Val Cys Phe Val Pro Thr Cys Ser Glu Ser Ser Pro Ser Cys Cys Gln
        115                 120                 125

Gln Ser Ser Cys Gln Pro Thr Cys Cys Thr Ser Ser Pro Cys Gln Gln
    130                 135                 140

Ala Cys Cys Val Pro Val Cys Ser Lys Ser Val Cys Tyr Val Pro Val
145                 150                 155                 160

Cys Ser Gly Ala Ser Thr Ser Cys Cys Gln Gln Ser Ser Cys Gln Pro
                165                 170                 175

Ala Cys Cys Thr Ala Ser Cys Cys Arg Pro Ser Ser Ser Val Ser Leu
            180                 185                 190

Leu Cys His Pro Val Cys Lys Ser Thr Cys Cys Val Pro Val Pro Ser
        195                 200                 205

Cys Gly Ala Ser Ala Ser Ser Cys Gln Pro Ser Cys Cys Arg Thr Ala
    210                 215                 220

Ser Cys Val Ser Leu Leu Cys Arg Pro Val Cys Ser Arg Pro Ala Cys
225                 230                 235                 240

Tyr Ser Leu Cys Ser Gly Gln Lys Ser Ser Cys
                245                 250

<210>  57
<211>  298
<212>  PRT
<213>  Homo sapiens

<400>  57

Met Ala Ala Ser Thr Met Ser Val Cys Ser Ser Ala Tyr Ser Asp Ser
1               5                   10                  15

Trp Gln Val Asp Asp Cys Pro Glu Ser Cys Cys Glu Pro Pro Cys Ser
            20                  25                  30

Ala Pro Ser Cys Cys Ala Pro Ala Pro Ser Leu Ser Leu Val Cys Thr
        35                  40                  45

Pro Val Ser Cys Val Ser Ser Pro Cys Cys Gln Ala Ala Cys Glu Pro
    50                  55                  60

Ser Ala Cys Gln Ser Gly Cys Thr Ser Ser Cys Thr Pro Ser Cys Cys
65                  70                  75                  80

Gln Gln Ser Ser Cys Gln Pro Ala Cys Cys Thr Ser Ser Pro Cys Gln
                85                  90                  95

Gln Ala Cys Cys Val Pro Val Cys Cys Lys Thr Val Cys Cys Lys Pro
            100                 105                 110

Val Cys Cys Val Pro Val Cys Cys Gly Ala Ala Ser Ser Cys Cys Arg
        115                 120                 125

Gln Ser Ser Cys Gln Pro Ala Cys Cys Ala Ser Ser Ser Cys Gln Pro
    130                 135                 140

Ala Cys Cys Val Pro Val Cys Cys Lys Pro Val Cys Cys Val Ser Thr
145                 150                 155                 160

Cys Ser Glu Asp Ser Ser Ser Cys Cys Gln Gln Ser Ser Cys Gln Pro
                165                 170                 175

Ala Cys Cys Thr Ser Ser Ser Tyr Gln Gln Ala Cys Cys Val Pro Val
            180                 185                 190

Cys Cys Lys Thr Val Tyr Cys Lys Pro Ile Cys Cys Val Pro Val Cys

195                 200                 205

Ser Arg Ala Ser Ser Ser Arg Cys Gln Gln Pro Ser Cys Gln Pro Ala
    210                 215                 220

Cys Cys Thr Thr Ser Cys Cys Arg Pro Ser Ser Ser Val Ser Leu Leu
225                 230                 235                 240

Cys His Pro Val Cys Arg Ser Thr Cys Cys Val Pro Val Ser Ser Cys
                245                 250                 255

Cys Ala Pro Thr Ser Ser Cys Gln Ser Ser Cys Cys Cys Pro Ala Ser
            260                 265                 270

Cys Val Ser Leu Leu Cys Arg Pro Ala Ser Ser Arg Leu Ala Cys Tyr
        275                 280                 285

Ser Leu Cys Ser Gly Lys Lys Ser Ser Cys
    290                 295

<210>  58
<211>  245
<212>  PRT
<213>  Homo sapiens

<400>  58

Met Ser Val Cys Ser Ser Asp Leu Ser Tyr Gly Ser Arg Val Cys Leu
1               5                   10                  15

Pro Gly Ser Cys Asp Ser Cys Ser Asp Ser Trp Gln Val Asp Asp Cys
            20                  25                  30

Pro Glu Ser Cys Cys Glu Pro Pro Cys Cys Ala Pro Ala Pro Cys Leu
        35                  40                  45

Ser Leu Val Cys Thr Pro Val Ser Arg Val Ser Ser Pro Cys Cys Arg
    50                  55                  60

Val Thr Cys Glu Pro Ser Pro Cys Gln Ser Gly Cys Thr Ser Ser Cys
65                  70                  75                  80

Thr Pro Ser Cys Cys Gln Gln Ser Ser Cys Gln Pro Ala Cys Cys Thr
                85                  90                  95

Ser Ser Pro Cys Gln Gln Ala Cys Cys Val Pro Val Cys Cys Lys Thr
            100                 105                 110

Val Cys Cys Lys Pro Val Cys Cys Met Pro Val Cys Cys Gly Pro Ser
        115                 120                 125

Ser Ser Cys Cys Gln Gln Ser Ser Cys Gln Pro Ala Cys Cys Ile Ser
    130                 135                 140

Ser Pro Cys Gln Gln Ser Cys Cys Val Pro Val Cys Cys Lys Pro Ile
145                 150                 155                 160

Cys Cys Val Pro Val Cys Ser Gly Ala Ser Ser Leu Cys Cys Gln Gln
                165                 170                 175

Ser Ser Cys Gln Pro Ala Cys Cys Thr Thr Ser Cys Cys Arg Pro Ser
            180                 185                 190

Ser Ser Val Ser Leu Leu Cys Arg Pro Val Cys Arg Pro Ala Arg Arg
        195                 200                 205

Val Pro Val Pro Ser Cys Cys Val Pro Thr Ser Ser Cys Gln Pro Ser
    210                 215                 220

Cys Gly Arg Leu Ala Ser Cys Gly Ser Leu Leu Cys Arg Pro Thr Cys
225                 230                 235                 240

Ser Arg Leu Ala Cys
                245

<210>  59
<211>  163
<212>  PRT
<213>  Homo sapiens

<400>  59

Met Ser Phe Asn Cys Ser Thr Arg Asn Cys Ser Ser Arg Pro Ile Gly
1               5                   10                  15

Gly Arg Cys Ile Val Pro Val Ala Gln Val Thr Thr Thr Ser Thr Thr
            20                  25                  30

Asp Ala Asp Cys Leu Gly Gly Ile Cys Leu Pro Ser Ser Phe Gln Thr
        35                  40                  45

Gly Ser Trp Leu Leu Asp His Cys Gln Glu Thr Cys Cys Glu Pro Thr
    50                  55                  60

Ala Cys Gln Pro Thr Cys Tyr Arg Arg Thr Ser Cys Val Ser Asn Pro
65                  70                  75                  80

Cys Gln Val Thr Cys Ser Arg Gln Thr Thr Cys Ile Ser Asn Pro Cys
                85                  90                  95

Ser Thr Thr Tyr Ser Arg Pro Leu Thr Phe Val Ser Ser Gly Cys Gln
            100                 105                 110

Pro Leu Gly Gly Ile Ser Ser Val Cys Gln Pro Val Gly Gly Ile Ser
        115                 120                 125

Thr Val Cys Gln Pro Val Gly Gly Val Ser Thr Val Cys Gln Pro Ala
    130                 135                 140

Cys Gly Val Ser Arg Thr Tyr Gln Gln Ser Cys Val Ser Ser Cys Arg
145                 150                 155                 160

Arg Thr Cys

<210> 60
<211> 96
<212> PRT
<213> Homo sapiens

<400> 60

Met Cys His Thr Ser Cys Ser Ser Gly Cys Gln Pro Ala Cys Cys Ala
1               5                   10                  15

Pro Ser Pro Cys Gln Ala Ser Cys Tyr Ile Pro Val Gly Cys Gln Ser
            20                  25                  30

Ser Val Cys Val Pro Val Ser Phe Lys Pro Ala Val Cys Val Pro Val
        35                  40                  45

Arg Cys Gln Ser Ser Val Cys Val Pro Val Ser Cys Arg Pro Val Val
    50                  55                  60

Tyr Ala Ala Pro Ser Cys Gln Ser Ser Gly Cys Cys Gln Pro Ser Cys
65                  70                  75                  80

Thr Ser Val Leu Cys Arg Pro Ile Ser Cys Ser Thr Pro Ser Cys Cys
                85                  90                  95

<210> 61
<211> 146
<212> PRT
<213> Homo sapiens

<400> 61

Met Cys His Thr Ser Cys Ser Ser Gly Cys Gln Pro Ala Cys Cys Ala
1               5                   10                  15

Pro Ser Pro Cys Gln Pro Ala Cys Cys Val Pro Ser Ser Cys Gln Ala
            20                  25                  30

Ser Cys Cys Val Pro Val Gly Cys Gln Ser Ser Val Cys Val Pro Val
        35                  40                  45

Ser Phe Lys Pro Ala Val Cys Leu Pro Val Ser Cys Gln Ser Ser Val
    50                  55                  60

Cys Val Pro Met Ser Phe Lys Ser Ala Val Cys Val Pro Val Ser Cys

65                  70                  75                  80

Gln Ser Ser Val Cys Val Pro Val Ser Cys Arg Pro Ile Val Cys Ala
                85                  90                  95

Ala Pro Ser Cys Gln Ser Ser Leu Cys Val Pro Val Ser Cys Arg Pro
            100                 105                 110

Val Val Tyr Ala Ala Pro Ser Cys Gln Ser Ser Gly Cys Cys Gln Pro
        115                 120                 125

Ser Cys Thr Ser Val Leu Cys Arg Pro Ile Ser Tyr Ser Ile Ser Ser
    130                 135                 140

Cys Cys
145

<210>  62
<211>  96
<212>  PRT
<213>  Homo sapiens

<400>  62

Met Cys His Thr Ser Cys Ser Pro Ala Cys Gln Pro Thr Cys Cys Ile
1               5                   10                  15

Arg Ser Pro Cys Gln Ala Ser Cys Tyr Val Pro Val Ser Cys Gln Ser
            20                  25                  30

Ser Val Cys Met Pro Val Ser Cys Thr Arg Ile Val Cys Val Ala Pro
        35                  40                  45

Ser Cys Gln Pro Ser Val Cys Val Pro Val Ser Cys Arg Pro Ile Ile
    50                  55                  60

Tyr Val Thr Pro Ser Cys Gln Ser Ser Gly Cys Cys Gln Pro Pro Cys
65                  70                  75                  80

Thr Thr Ala Leu Cys Arg Pro Ile Ser Cys Ser Thr Pro Ser Cys Cys
                85                  90                  95

<210>  63
<211>  112
<212>  PRT
<213>  Homo sapiens

<400>  63

Met Cys His Thr Ser His Ser Ser Gly Cys Pro Met Ala Cys Pro Gly
1               5                   10                  15

Ser Pro Cys Cys Val Pro Ser Thr Cys Tyr Pro Pro Glu Gly Tyr Gly
            20                  25                  30

Thr Ser Cys Cys Cys Ser Ala Pro Cys Val Ala Leu Leu Cys Arg Pro
        35                  40                  45

Leu Cys Gly Val Ser Thr Cys Cys Gln Pro Ala Cys Cys Val Pro Ser
    50                  55                  60

Pro Cys Gln Val Ala Cys Cys Val Pro Val Ser Cys Lys Pro Val Leu
65                  70                  75                  80

Cys Val Ala Ser Phe Cys Pro Thr Ser Gly Cys Cys Gln Pro Phe Cys
                85                  90                  95

Pro Thr Leu Val Tyr Arg Pro Val Thr Trp Ser Thr Pro Thr Gly Cys
            100                 105                 110

<210>  64
<211>  172
<212>  PRT
<213>  Homo sapiens

<400>  64

Met Ser Tyr Asn Cys Cys Ser Gly Asn Phe Ser Ser Arg Ser Cys Gly
1               5                   10                  15

Gly Tyr Leu His Tyr Pro Ala Ser Ser Cys Gly Phe Ser Tyr Pro Ser
            20                  25                  30

Asn Gln Val Tyr Ser Thr Asp Leu Cys Ser Pro Ser Thr Cys Gln Leu
        35                  40                  45

Gly Ser Ser Leu Tyr Arg Gly Cys Gln Gln Thr Cys Trp Glu Pro Thr
    50                  55                  60

Ser Cys Gln Thr Ser Tyr Val Glu Ser Ser Pro Cys Gln Thr Ser Cys
65                  70                  75                  80

Tyr Arg Pro Arg Thr Ser Leu Leu Cys Ser Pro Cys Gln Thr Thr Tyr
                85                  90                  95

Ser Gly Ser Leu Gly Phe Gly Ser Ser Ser Cys Arg Ser Leu Gly Tyr
            100                 105                 110

Gly Ser Arg Ser Cys Tyr Ser Val Gly Cys Gly Ser Ser Gly Phe Arg
        115                 120                 125

Ser Leu Gly Tyr Gly Gly Cys Gly Phe Pro Ser Leu Gly Tyr Gly Val
    130                 135                 140

Gly Phe Cys Arg Pro Thr Tyr Leu Ala Ser Arg Ser Cys Gln Ser Ser
145                 150                 155                 160

Cys Tyr Arg Pro Thr Cys Gly Ser Gly Phe Tyr Tyr
                165                 170

<210>  65
<211>  105
<212>  PRT
<213>  Homo sapiens

<400>  65

Met Gly Cys Cys Pro Gly Asp Cys Phe Thr Cys Cys Thr Gln Glu Gln
1               5                   10                  15

Asn Cys Cys Glu Glu Cys Cys Cys Gln Pro Gly Cys Cys Gly Cys Cys
            20                  25                  30

Gly Ser Cys Cys Gly Cys Gly Gly Ser Gly Cys Gly Gly Ser Gly Cys
        35                  40                  45

Gly Gly Ser Cys Cys Gly Ser Ser Cys Cys Gly Ser Gly Cys Gly Gly
    50                  55                  60

Cys Gly Gly Cys Gly Gly Cys Gly Gly Gly Cys Cys Gly Ser Ser Cys
65                  70                  75                  80

Cys Gly Ser Ser Cys Cys Gly Ser Gly Cys Cys Gly Pro Val Cys Cys
                85                  90                  95

Gln Pro Thr Pro Ile Cys Asp Thr Lys
            100                 105

<210>  66
<211>  88
<212>  PRT
<213>  Homo sapiens

<400>  66

His Tyr Gly Ser Tyr Tyr Gly Gly Leu Gly Tyr Ser Cys Gly Gly Phe
1               5                   10                  15

Gly Gly Leu Gly Tyr Gly Tyr Gly Cys Gly Cys Gly Ser Phe Cys Arg
            20                  25                  30

Arg Gly Ser Gly Cys Gly Tyr Gly Gly Tyr Gly Tyr Gly Ser Gly Phe
        35                  40                  45

Gly Ser Tyr Gly Tyr Gly Ser Gly Phe Gly Gly Tyr Gly Tyr Gly Ser
    50                  55                  60

Gly Phe Gly Gly Tyr Gly Tyr Gly Cys Cys Arg Pro Ser Tyr Asn Gly
65                  70                  75                  80

Gly Tyr Gly Phe Ser Gly Phe Tyr
                85